# EUROPÄISCHE PATENTSCHRIFT

(11) **EP 1 633 470 B1**
(45) Veröffentlichungstag und Bekanntmachung des Hinweises auf die Patenterteilung: **21.07.2010**
(21) Anmeldenummer: 04739696.5
(22) Anmeldetag: 08.06.2004
(51) Int. Cl.: B01J 13/02, B01J 13/04, C11D 3/50

(54) **LAGERSTABILE BLEICHMITTELZUSAMMENSETZUNGEN AUF BASIS VON PEROXYCARBONS UREN**
PERCARBOXYLIC ACID-BASED BLEACH COMPOSITIONS HAVING A LONG SHELF LIFE
COMPOSITIONS D'AGENTS DE BLANCHIMENT A BASE D'ACIDES PEROXYCARBONIQUES PRESENTANT UNE BONNE STABILITE AU STOCKAGE

(30) Priorität: 13.06.2003 DE 10327127; 22.12.2003 DE 10361084
(43) Veröffentlichungstag der Anmeldung: 15.03.2006
(73) Patentinhaber: Henkel AG & Co. KGaA, 40589 Düsseldorf (DE)
(72) Erfinder: SCHMIEDEL, Peter, 40599 Düsseldorf (DE); BARRELEIRO, Paula, 40724 Hilden (DE); VON RYBINSKI, Wolfgang, 40593 Düsseldorf (DE); ORLICH, Bernhard, 08005 Barcelona (DE)
(86) Internationale Anmeldenummer: PCT/EP2004/006167
(87) Internationale Veröffentlichungsnummer: WO 2004/110611

(56) Entgegenhaltungen:
- EP-A- 0 653 485
- WO-A-02/24319
- WO-A-03/045545
- US-A- 5 597 791

## Beschreibung

Die vorliegende Erfindung betrifft ein Verfahren zur Herstellung von mit mindestens einer organischen Peroxycarbonsäure beladenen Gelkapseln sowie die auf diese Weise hergestellten Gelkapseln selbst. Darüber hinaus betrifft die vorliegende Erfindung die Verwendung dieser Gelkapseln als Bleichmittel bzw. Bleichmittelkomponente, insbesondere für ihren Einsatz in Wasch- und Reinigungsmitteln, insbesondere in flüssigen Wasch- und Reinigungsmitteln, Zahnpflegemitteln, Haarfärbemitteln und Entfärbungs- bzw. Bleichmittelzusammensetzungen für technische Anwendungen sowie die diese Gelkapseln enthaltenden Produkte selbst, d. h. Wasch- und Reinigungsmittel, insbesondere flüssige Wasch- und Reinigungsmittel, Zahnpflegemittel, Haarfärbemittel und Entfärbungs- bzw. Bleichmittelzusammensetzungen für technische Anwendungen, welche die erfindungsgemäßen Gelkapseln enthalten.

Bei flüssigen, insbesondere wasserhaltigen Wasch- und Reinigungsmitteln, die sich aufgrund ihrer positiven Produkteigenschaften, wie einer besseren und schnelleren Löslichkeit und Anwendbarkeit, einer zunehmenden Beliebtheit erfreuen, ist die Einformulierung bzw. Inkorporierung von Bleich(mittel)komponenten aus zahlreichen Gründen problematisch. So verlieren eingesetzte Bleichmittel aufgrund von Zersetzungs- bzw. Hydrolysereaktionen und Inkompatibilitäten gegenüber anderen Bestandteilen der Waschmittelformulierung, wie z. B. Enzymen oder Tensiden, häufig schon bei der Lagerung oder aber im Rahmen der Anwendung der Produkte ihre Aktivität. Als nachteilige Folge verliert die Waschmittelformulierung hierdurch deutlich an Waschleistung, insbesondere Bleichvermögen, so daß insbesondere bleichbare Verschmutzungen nicht mehr zufriedenstellend entfernt werden können.

Die üblicherweise für feste Waschmittelformulierungen verwendeten Bleichkomponenten, wie beispielsweise Perborate oder Percarbonate, sind äußerst feuchtigkeitsempfindlich, so daß sie in einem flüssigen und insbesondere wasserhaltigen Wasch- oder Reinigungsmittel aufgrund des Verlustes von Aktivsauerstoff häufig innerhalb weniger Tage ihre Bleichwirkung verlieren.

Demgegenüber sind Peroxycarbonsäuren, insbesondere Imidoperoxycarbonsäuren, deren wichtigster Vertreter Phthalimidoperoxycapronsäure (PAP) ist, effizienter und weniger hydrolyseempfindlich und im Stand der Technik als Bleichmittel für Wasch- und Reinigungsmittel bekannt. Jedoch ist ihre Lagerstabilität bei weitem nicht ausreichend, um eine langfristige Einsetzbarkeit des entsprechenden Wasch- oder Reinigungsmittels ohne einhergehenden Aktivitätsverlust zu gewährleisten. Besonders problematisch ist daher der Einsatz von Peroxycarbonsäuren, insbesondere Imidoperoxycarbonsäuren, in flüssigen Wasch- und Reinigungsmitteln.

Aufgrund der Nachteile, die sich in bezug auf eine Veränderung der Wasch- oder Reinigungsmittelformulierung infolge des Abbaus von Imidoperoxycarbonsäuren, insbesondere PAP, ergeben, wurde im Stand der Technik versucht, die Imidoperoxycarbonsäuren (z. B. PAP) wirkungsvoll zu verkapseln, so daß die Imidoperoxycarbonsäure mit den übrigen Komponenten der Wasch- bzw. Reinigungsmittelformulierung nicht in Kontakt geraten kann.

So beschreibt die EP 0 510 761 B1 bzw. die zu derselben Patentfamilie gehörende US-A-5 230 822 ein Verfahren zur Verkapselung von Waschmittelzusatzstoffen im allgemeinen, wie z. B. Enzyme, Bleichmittel, unter anderem auch PAP, Bleichmittelvorläufer und Bleichkatalysatoren, wobei als Schutzhülle zur Verkapselung ein Wachs verwendet wird, dessen Schmelzpunkt zwischen 40 °C und 50 °C liegt. Die Herstellung der wachsbeschichteten Partikel erfolgt dabei über ein Aufsprühen des geschmolzenen Wachses. Dabei muß das Wachs zunächst auf Temperaturen oberhalb seines Schmelzpunktes erhitzt werden, was mitunter in bezug auf die zu verkapselnden Substanzen nachteilig sein kann. Dieses Verfahren weist zudem den Nachteil auf, daß die Wirksubstanz erst bei Temperaturen oberhalb des Schmelzpunktes des verwendeten Wachses - also erst oberhalb von Temperaturen zwischen 40 °C und 50 °C - freigesetzt wird, was insbesondere heutigen Verbraucher- bzw. Anwenderanforderungen nicht gerecht wird, da - vor dem Hintergrund der Entwicklung leistungsfähiger Wasch- und Reinigungsmittelformulierungen und der Einsparung von Energiekosten - häufig auch bei niedrigeren Temperaturen, insbesondere bei etwa 30 °C, gewaschen werden soll. Weiterhin weist ein Wachs mit einem hohen Schmelzpunkt den Nachteil auf, daß es insbesondere bei niedrigeren Temperaturen Rückstände auf der Wäsche verursacht, da es bei diesen Temperaturen nicht vollständig emulgiert wird.

Die EP 0 653 485 A1 betrifft wirkstoffhaltige Kapselzusammensetzungen, die unter anderem Bleichmittel, wie z. B. PAP, enthalten können und bei denen der Wirkstoff im Kapselinneren als Dispersion in Öl vorliegt. Die Herstellung dieser Kapseln, deren Hülle aus hydrophilen, erst während des Waschvorgangs bzw. der Anwendung löslich werdenden Polymeren gebildet wird, erfordert einen aufwendigen und technologisch nicht einfach durchzuführenden Emulgierprozeß.

Die WO 93/045545 A1 beschreibt wirkstoffhaltige Kapseln, die unter Verwendung einer Trägerphase und eines Blockcopolymers mit Hilfe eines Miniemulsionsverfahrens hergestellt werden. Die dort angeführten Wirkstoffe müssen in der Ölphase gelöst sein, so daß die dort beschriebenen Kapselsysteme weitgehend auf in der Ölphase lösliche, insbesondere hydrophobe Aktiv- bzw. Wirkstoffe beschränkt sind. Bleichmittel sind dort nicht erwähnt. Zur Herstellung der Miniemulsion ist eine Hochdruckhomogenisation erforderlich.

Vor diesem Hintergrund besteht somit eine Aufgabe der vorliegenden Erfindung darin, Peroxycarbonsäuren, insbesondere Imidoperoxycarbonsäuren, wie Phthalimidoperoxycapronsäure (PAP), in lagerstabiler Form bereitzustellen.

Eine weitere Aufgabe besteht darin, eine Einlagerung oder Verkapselung bzw. Beschichtung von Peroxycarbonsäuren, insbesondere Imidoperoxycarbonsäuren, wie Phthalimidoperoxycapronsäure (PAP), mit gegenüber dem Stand der Technik verbesserten Eigenschaften sowie ein entsprechendes Herstellungsverfahren bereitzustellen.

Noch eine weitere Aufgabe der vorliegenden Erfindung ist die Bereitstellung von Gelkapseln, die mit Peroxycarbonsäuren in fester Form beladen sind und die zu einer guten Stabilisierung der Peroxycarbonsäuren und damit zu einer verbesserten Lagerstabilität führen. Insbesondere soll im Rahmen der vorliegenden Erfindung eine Verkapselung bereitgestellt werden, die insbesondere während des Waschvorganges bereits bei niedrigen Temperaturen weitgehend rückstandslos aufgelöst bzw. solubilisiert wird, so daß die Freisetzung der Peroxycarbonsäure nicht behindert wird und gleichzeitig Rückstände auf der Wäsche vermieden werden. Insbesondere soll ein solches Verfahren es ermöglichen, mit mindestens einer Peroxycarbonsäure als Wirksubstanz beladene Gelkapseln herzustellen, welche die zuvor geschilderten Nachteile des Standes der Technik zumindest weitgehend vermeiden. Dabei soll in bezug auf die Gelkapselherstellung gewährleistet sein, daß möglichst wenig Ausgangssubstanz(en) zum Aufbau der Gelkapselhülle eingesetzt wird bzw. werden, so daß ein hoher Wirkstoffgehalt realisierbar ist.

Die Anmelderin hat nun überraschenderweise herausgefunden, daß sich organische Peroxycarbonsäuren, wie Imidoperoxycarbonsäuren (z. B. PAP), durch eine Gelkapselmatrix auf Basis einer verfestigten bzw. gelierten Ölphase stabilisieren lassen.

Gegenstand der vorliegenden Erfindung gemäß einem ersten Aspekt ist somit ein Verfahren zur Herstellung von mit mindestens einer organischen Peroxycarbonsäure, insbesondere Imidoperoxycarbonsäure, beladenen Gelkapseln, bei dem mindestens eine organische Peroxycarbonsäure, insbesondere Imidoperoxycarbonsäure, in Form fester Teilchen in eine Gelmatrix auf Basis einer gegenüber der organischen Peroxycarbonsäure inerten, durch Zugabe mindestens eines Stabilisators, insbesondere Gelbildners, verfestigten und/oder gelierten Ölphase, wobei der Schmelzpunkt der Ölphase bei Atmosphärendruck unterhalb von 35 °C liegt, eingelagert wird, insbesondere mittels einer die Peroxycarbonsäure umgebenden Verkapselung und/oder Beschichtung.

Erfindungsgemäß resultieren somit Gelkapseln, welche Kapselhüllen auf Basis einer Gelmatrix und Kapselkerne, welche mindestens eine organische Peroxycarbonsäure umfassen, aufweisen. Die erfindungsgemäßen Gelkapseln können auch jeweils mehrere Kapselkerne aufweisen, insbesondere können sich im Rahmen der Herstellung Agglomerate aus Peroxycarbonsäureteilchen bilden. Eine Agglomeration mehrerer Gelkapseln ist ebenfalls möglich. Auf diese Weise kann sozusagen eine Matrix entstehen, in welche mehrere Kapselkerne eingebettet bzw. eingelagert sind.

Insbesondere betrifft die vorliegende Erfindung ein Verfahren zur Herstellung von mit mindestens einer organischen Peroxycarbonsäure, insbesondere Imidoperoxycarbonsäure, beladenen Gelkapseln, welches die folgenden Verfahrensschritte umfaßt:
(a) Bereitstellung einer gegenüber der organischen Peroxycarbonsäure inerten, durch Zugabe eines Stabilisators, insbesondere Gelbildners, gelierbaren bzw. verfestigbaren Ölphase, deren Schmelzpunkt bei Atmosphärendruck (101325 Pa) unterhalb von 35 °C liegt; dann
(b) Inkontaktbringen der Ölphase mit mindestens einem Stabilisator, insbesondere Gelbildner, vorzugsweise bei einer Temperatur oberhalb der Gelbildungstemperatur, so daß ein vorzugsweise flüssiges Matrixmedium auf Basis der unter Verfahrensschritt (a) bereitgestellten Ölphase und des Stabilisators, insbesondere Gelbildners, erhalten wird; dann
(c) gegebenenfalls Abkühlen des in Verfahrensschritt (b) erhaltenen Matrixmediums, vorzugsweise auf Temperaturen oberhalb der Gelbildungstemperatur des Matrixmediums; dann
(d) Einbringen mindestens einer zu stabilisierenden organischen Peroxycarbonsäure in Form fester Teilchen in das in Verfahrensschritt (b) erhaltene, gegebenenfalls in Verfahrensschritt (c) auf Temperaturen vorzugsweise oberhalb der Gelbildungstemperatur temperierte, vorzugsweise flüssige Matrixmedium, so daß eine Dispersion der festen Peroxycarbonsäure in dem vorzugsweise flüssigen Matrixmedium resultiert; dann
(e) Einbringen des in Verfahrensschritt (d) erhaltenen Matrixmediums mit den darin eingebrachten, dispergierten, festen Teilchen der zu stabilisierenden organischen Peroxycarbonsäure in ein Dispersionsmittel, insbesondere Wasser, vorzugsweise bei Temperaturen unterhalb der Gelbildungstemperatur und/oder vorzugsweise unter Zerkleinern, insbesondere durch Eintragen von Scherkräften, so daß in vorzugsweise wäßriger Dispersion befindliche Gelkapseln auf Basis von mindestens einer in eine Ölphase/Stabilisator-Gelmatrix eingelagerten oder hiermit verkapselten bzw. beschichteten organischen Peroxycarbonsäure resultieren, wobei gegebenenfalls durch das Zerkleinern, insbesondere durch Eintragen von Scherkräften, die Gelkapselgröße gezielt eingestellt werden kann; dann
(f) gegebenenfalls Entfernen des Dispersionsmittels, insbesondere Wasser, und etwaiger überschüssiger Ölphase aus der in Verfahrensschritt (e) erhaltenen Gelkapseldispersion, insbesondere durch Zentrifugieren und/oder Filtrieren; anschließend
(g) gegebenenfalls Trocknen der auf diese Weise erhaltenen Gelkapseln.

Bei dem erfindungsgemäßen Verfahren werden als zu stabilisierende, insbesondere zu verkapselnde bzw. zu beschichtende Substanzen organische Peroxycarbonsäuren verwendet. Letztere können aus organischen Mono- und Diperoxycarbonsäuren ausgewählt sein. Beispiele sind insbesondere Dodekandiperoxysäure oder vorzugsweise Imidoperoxysäuren, besonders bevorzugt 6-Phthalimidoperoxycapronsäure (6-Phthalimidoperoxyhexansäure, PAP). Dabei sollte die Peroxycarbonsäure vorteilhafterweise bei Atmosphärendruck (101325 Pa) einen Schmelzpunkt oberhalb von 25 °C, insbesondere oberhalb von 35 °C, vorzugsweise oberhalb von 45 °C, bevorzugt oberhalb von 50 °C, besonders bevorzugt oberhalb von 100 °C, aufweisen; hierdurch ist gewährleistet, daß die verwendete Peroxycarbonsäure im wesentlichen in Form fester Teilchen vorliegt, so daß ein Abbau der Peroxycarbonsäure während der Gelkapselherstellung im Rahmen des erfindungsgemäßen Verfahrens zumindest weitgehend vermieden bzw. verringert wird.

Im allgemeinen sollten die weiteren Komponenten, welche in dem erfindungsgemäßen Verfahren zur Herstellung der Gelkapseln verwendet werden, derart ausgewählt sein, daß sie zumindest im wesentlichen kompatibel in bezug auf die zu verkapselnde bzw. zu beschichtende Peroxycarbonsäure sind, d. h. es sollten keine unerwünschten chemischen Reaktionen, wie insbesondere Abbau-, Oxidations- bzw. Reduktionsreaktionen und/oder Hydrolysereaktionen, zwischen diesen Komponenten und der Peroxycarbonsäure und keine durch die weiteren Komponenten induzierten Reaktionen der Peroxycarbonsäure auftreten, welche zu ihrem Abbau, insbesondere Aktivitätsverlust, führen.

Im Rahmen des erfindungsgemäßen Verfahrens kann die in Verfahrensschritt (a) bereitgestellte gelierbare bzw. verfestigbare Ölphase insbesondere ausgewählt sein aus der Gruppe von Paraffinölen, Isoparaffinölen, Silikonölen, Glyceriden, Triglyceriden, naphthalinhaltigen Ölen, kohlenwasserstoffhaltigen Lösemitteln und deren Mischungen, pflanzlichen Ölen, Rizinusöl, Maisöl, Erdnußöl, Alkylpalmitaten und Alkylalkoholbenzoaten. Insbesondere werden erfindungsgemäß Paraffinöle bevorzugt. Darüber hinaus sollte der Schmelzpunkt der gelierbaren bzw. verfestigbaren Ölphase (d. h. der reinen, d. h. nichtgelierten bzw. nichtverfestigten Ölphase) bei Atmosphärendruck ≤ 30 °C, insbesondere ≤ 25 °C und vorzugsweise ≤ 20 °C sein, so daß die gelierbare bzw. verfestigbare Ölphase im Rahmen des erfindungsgemäßen Verfahrens in flüssiger Form vorliegt. D. h. mit anderen Worten, daß die erfindungsgemäß eingesetzte reine Ölphase bei Temperaturen oberhalb von 35 °C, insbesondere oberhalb von 30 °C, vorzugsweise oberhalb von 25 °C , bevorzugt oberhalb von 20 °C, in flüssiger Form vorliegt, so daß sie in einem Waschprozeß von den übrigen Wasch- bzw. Reinigungsmittelbestandteilen ohne weiteres solubilisiert bzw. dispergiert werden kann. In diesem Zusammenhang wird unter einer gelierbaren bzw. verfestigbaren Ölphase eine solche Ölphase verstanden, die mit einem Stabilisator, insbesondere Gelbildner, ein Gel bzw. eine Gelmatrix ausbilden kann bzw. die mit einem Stabilisator zu einer verfestigten (z. B. zu einer chemisch und/oder physikalisch vernetzten) Struktur führen kann. Erfindungsgemäß besonders geeignet sind Paraffine mit einem breiten Schmelzbereich und Anteilen, die auch unterhalb von 30 °C flüssig vorliegen, da diese in einem Waschprozeß leichter dispergierbar sind.

Der im erfindungsgemäßen Verfahren verwendete Stabilisator, insbesondere Gelbildner, kann ein Blockcopolymer sein. Dabei kann das Blockcopolymer ein insbesondere hydrophobes, mit der gelierbaren bzw. verfestigbaren Ölphase unterhalb der entsprechenden Gelbildungstemperatur ein Gel bzw. eine Ölphase/Stabilisator-Gelmatrix, insbesondere Organogel, ausbildendes organisches Blockcopolymer sein. Als Stabilisatoren, insbesondere Gelbildner, eignen sich auch Metallseifen, Alkylhydroxybutyramide und Schichtsilikate. Erfindungsgemäß können weiterhin auch die in den Artikeln Terech et al., Chem. Rev., Nr. 97, Seiten 3133 bis 3159 (1997) und Abdallah et al., Adv. Mater., Nr. 12, N 17, Seiten 1237 bis 1247 (2000), deren gesamter Offenbarungsgehalt hiermit unter Bezugnahme eingeschlossen ist, aufgeführten Verbindungen als Stabilisatoren bzw. Gelbildner eingesetzt werden. Beispielsweise kann der Stabilisator bzw. Gelbildner ausgewählt sein aus Fettsäurederivaten, Fettalkoholen, Steroidderivaten, Anthrylderivaten, Aminosäurederivaten, Organometallverbindungen und Dibenzyliden-D-sorbitol (DBS).

Im Rahmen der vorliegenden Erfindung versteht man unter Gelen insbesondere Organogele in Form von formbeständigen, leicht deformierbaren, an Flüssigkeiten reichen dispersen Systeme, die aus mindestens zwei Komponenten bestehen, nämlich insbesondere der gelierbaren bzw. verfestigbaren Ölphase einerseits, die insbesondere als Dispersionsmittel fungiert, und dem Stabilisator, insbesondere Blockcopolymer, als Gelbildner bzw. Geliermittel andererseits.

Ohne sich auf eine spezielle Theorie festlegen zu wollen, verläuft die Ausbildung der Gelmatrix in Verfahrensschritt (e) aufgrund physikalischer Wechselwirkungen zwischen der Ölphase und dem Stabilisator, insbesondere Gelbildner. Insbesondere bildet dabei der Stabilisator, insbesondere Gelbildner, im Dispersionsmittel, d. h. in der Ölphase, ein räumliches Netzwerk aus, wobei die Teilchen an verschiedenen Stellen aneinanderhaften, so daß aufgrund von im wesentlichen physikalischen Wechselwirkungen ein Verbund gebildet wird. Die erfindungsgemäß hergestellten Gele bzw. Gelkapseln besitzen eine Fließgrenze und sind insbesondere elastisch und/oder plastisch verformbar. Unterhalb einer für das jeweilige Gel charakteristischen Gelbildungstemperatur T_{gel}, die auch als Gelierungstemperatur bezeichnet wird, bildet dieser Verbund aus Gelbildner und Dispersionsmittel (= Ölphase) eine gelartige Struktur aus, wohingegen er sich bei Temperaturen oberhalb dieser Gelbildungstemperatur verflüssigt.

Bei dem erfindungsgemäß als Stabilisator bzw. Gelbildner eingesetzten Blockcopolymer handelt es sich insbesondere um ein Copolymer mit gelierenden Eigenschaften gegenüber der im erfindungsgemäßen Verfahren verwendeten gelierbaren bzw. verfestigbaren Ölphase.

Das erfindungsgemäß verwendete Blockcopolymer kann ein aus mindestens zwei Blöcken oder Komponenten A und B bestehendes Polymer (A-B-...)ₙ (n = Anzahl der sich wiederholenden Einheiten) sein, bei dem mindestens einer der Blöcke ein harter Block und mindestens ein anderer der Blöcke ein weicher Block ist, die sich folglich im Verhältnis zueinander durch ihre Härte, welche insbesondere durch ihre Glasübergangstemperaturen bestimmt wird, unterscheiden.

Das erfindungsgemäß verwendete Blockpolymer kann ein aus mindestens zwei Blöcken oder Komponenten bestehendes Polymer sein. Dabei kann mindestens einer der Blöcke ein harter Block und mindestens ein anderer der Blöcke ein weicher Block sein. Dabei sollten sich die Glasübergangstemperaturen des harten und des weichen Blockes um mindestens 50 °C, insbesondere um mindestens 60 °C, vorzugsweise um mindestens 70 °C unterscheiden. Beispielsweise kann der harte Block eine Glasübergangstemperatur T_{g(hart)} > 20 °C, insbesondere T_{g(hart)} > 50 °C, bevorzugt T_{g(hart)} > 90 °C, aufweisen, während der weiche Block eine Glasübergangstemperatur T_{g(weich)} ≤ 20 °C, insbesondere T_{g(weich)} ≤ 0 °C, bevorzugt T_{g(weich)} ≤ -45 °C, aufweisen kann.

Mindestens ein Block des Blockcopolymers, vorzugsweise der harte Block, sollte nicht oder nur schlecht öllöslich sein oder allenfalls mäßig öllöslich sein, während mindestens ein anderer Block des Blockcopolymers, vorzugsweise der weiche Block, öllöslich ausgebildet sein sollte. Insbesondere sollte mindestens ein Block des Blockcopolymers, vorzugsweise der harte Block, schlechter öllöslich als mindestens ein anderer Block des Blockcopolymers, vorzugsweise der weiche Block, sein.

Der harte Block des Blockcopolymers kann vorzugsweise ausgewählt sein aus der Gruppe von Polystyrolen, Poly(meth)acrylaten, Polycarbonaten, Polyestern, Polyanilinen, Poly-p-phenylenen, Polysulfonethern, Polyacrylnitrilen, Polyamiden, Polyimiden, Polyethern, Polyvinylchloriden und deren Mischungen. Der weiche Block des Blockcopolymers kann ausgewählt sein aus der Gruppe von Kautschuken, insbesondere gegebenenfalls substituierten Polyalkylenen, vorzugsweise Polybutadienen, und Mischungen von Kautschuken oder Polyalkylenen, wie Polybutadien-Ethylen, Polybutadien-Propylen, Polyethylenethylenen; Polyvinylalkoholen; Polyalkylenglykolen, wie Polyethylenglykolen und Polypropylenglykolen; Polydimethoxysiloxanen und Polyurethanen.

Insbesondere sind die mindestens zwei Blöcke oder Komponenten des Blockcopolymers jeweils vom Typ (Styrol/α-Olefin), wobei die α-Olefine der zwei Blöcke eine unterschiedliche Anzahl an Kohlenstoffatomen aufweisen können. Insbesondere ist das Blockcopolymer ausgewählt aus der Gruppe von Poly-(Styrol-Ethylen/Butylen-Styrol) und/oder Poly-(Styrol-Ethylen/Propylen-Styrol). Insbesondere kann das Blockcopolymer ein Styrol/Butadien-Blockcopolymer, Styrol/Butylen-Blockcopolymer, Styrol/Propylen-Blockcopolymer, Styrol/Butylen-Propylen-Blockcopolymer oder Styrol/Kautschuk-Blockcopolymer sein. Erfindungsgemäß verwendbare Styrol/Kautschuk-Blockcopolymere sind beispielsweise kommerziell unter der Bezeichnung Kraton^{®}, z. B. Kraton^{®} G-1650 und Kraton^{®} G-1651 der Firma Kraton Polymers erhältlich. Die Blöcke der vorgenannten Blockcopolymere können dabei als beispielsweise als Dreiblockcopolymere, sternförmig oder radial verzweigte Copolymere, Multiblöcke von Randompolymeren oder Propfcopolymere angeordnet sein.

Im Rahmen des erfindungsgemäßen Verfahrens erfolgt das Inkontaktbringen der gelierbaren bzw. verfestigbaren Ölphase mit dem Stabilisator, insbesondere Gelbildner, in Verfahrensschritt (b) vorzugsweise unter Rühren und/oder bei Temperaturen oberhalb der Gelbildungstemperatur, insbesondere bei Temperaturen von 50 °C bis 100 °C, vorzugsweise von 60 °C bis 80 °C, bevorzugt bei etwa 70 °C, so daß ein flüssiges Matrixmediums auf Basis der unter Verfahrensschritt (a) bereitgestellten Ölphase und des Stabilisators, insbesondere Gelbildners, erhalten wird. Anschließend erfolgt gegebenenfalls ein Abkühlen des in Verfahrensschritt (b) erhaltenen flüssigen Matrixmediums, vorzugsweise auf Temperaturen oberhalb der Gelbildungstemperatur, insbesondere auf Temperaturen von 25 °C bis 50 °C, vorzugsweise von 35 °C bis 45 °C, bevorzugt auf etwa 40 °C.

Das Abkühlen des flüssigen Matrixmediums in Verfahrensschritt (c) auf Temperaturen oberhalb der Gelbildungstemperatur erfolgt vor dem Hintergrund, einerseits eine Gelbildung vor dem Einbringen der zu stabilisierenden Peroxycarbonsäure zu verhindern und andererseits eine gegebenenfalls temperaturabhängige Zersetzung, insbesondere aufgrund chemischer Abbau-, Oxidations- bzw. Reduktionsreaktionen und/oder Hydrolysereaktionen bzw. durch gegebenenfalls temperaturabhängiges Auflösen, der zu stabilisierenden Peroxycarbonsäure zumindest im wesentlichen zu unterbinden.

Die in Verfahrensschritt (d) in das vorzugsweise flüssige Matrixmedium eingebrachte, zu stabilisierende organische Peroxycarbonsäure in Form fester Teilchen weist eine Teilchengröße bzw. einen Teilchendurchmesser ≤ 3.000 µm, insbesondere ≤ 2.500 µm, vorzugsweise ≤ 2.250 µm, bevorzugt ≤ 2.000 µm, besonders bevorzugt ≤ 1.500 µm, auf. In diesem Zusammenhang sollte die Teilchengröße der organische Peroxycarbonsäure 10 bis 3.000 µm, insbesondere 50 bis 2.500 µm, vorzugsweise 100 bis 1.500 µm, betragen. Dabei kann erfindungsgemäß ein Einstellen der Teilchengröße der festen Peroxycarbonsäurepartikel und der damit in Zusammenhang stehenden Gelkapselgröße in Verfahrensschritt (e) durch Zerkleinern, z. B. Rühren, Vibration, Ultraschalleintrag und/oder Eintrag von Scherkräften, erfolgen, so daß eine gezielte Anpassung der Teilchen- bzw. Gelkapselgröße entsprechend ihrer jeweiligen späteren Verwendung möglich ist.

Das in Verfahrensschritt (e) verwendete Dispersionsmittel kann eine Substanz sein, die imstande ist, das in Verfahrensschritt (d) erhaltene Matrixmedium mit den darin eingebrachten, insbesondere dispergierten festen Teilchen der zu stabilisierenden organischen Peroxycarbonsäure zu dispergieren. Vorzugsweise handelt es sich bei dem in Verfahrensschritt (e) eingebrachten Dispersionsmittel um eine polare Substanz, insbesondere Wasser. Möglich ist aber auch die Verwendung von Glycerin als Dispersionsmittel, gegebenenfalls in Mischung mit Wasser.

Dabei kann in Verfahrensschritt (e) das Dispersionsmittel in das Matrixmedium als auch das Matrixmedium in das Dispersionsmittel eingebracht werden.

In Verfahrensschritt (e) liegt die Temperatur vorzugsweise unterhalb der Gelbildungstemperatur. Sie wird insbesondere derart ausgewählt, daß das Matrixmedium mit den darin eingebrachten, insbesondere dispergierten festen Teilchen der zu stabilisierenden organischen Peroxycarbonsäure noch fließfähig ist. Insbesondere sollte dabei gewährleistet sein, daß die Temperatur derart ausgewählt wird, daß eine temperaturabhängige Zersetzung bzw. Auflösung der zu stabilisierenden organischen Peroxycarbonsäuren vermieden wird.

Bei dem erfindungsgemäßen Verfahren kann in Verfahrensschritt (e) dem Dispersionsmittel, insbesondere Wasser, mindestens ein Dispergiermittel (Dispergator), insbesondere ein tensidisches Dispergiermittel, vorzugsweise ein kationisches und/oder anionisches Tensid zugegeben werden. Das Dispergiermittel sollte zumindest im wesentlichen keine Halogenidionen, insbesondere keine Chloridionen enthalten. Insbesondere sollte die Menge an Halogenidionen der in Verfahrensschritt (e) erhaltenen Dispersion der festen Peroxycarbonsäure in dem vorzugsweise flüssigen Matrixmedium höchstens 500 ppm, insbesondere höchstens 100 ppm, vorzugsweise höchstens 30 ppm, betragen, da die zu stabilisierende Peroxycarbonsäure durch die Anwesenheit von Halogenid, insbesondere Chlorid - wie nachfolgend noch detailliert ausgeführt - schneller abgebaut wird. Das kationische Tensid kann bei dem erfindungsgemäßen Verfahren beispielsweise ausgewählt werden aus der Gruppe von quartären Ammoniumverbindungen, wie Dimethyldistearylammoniumsalzen; Esterquats, insbesondere quarternierten Fettsäuretrialkanolaminestersalzen; Salzen langkettiger primärer Amine; quaternären Ammoniumverbindungen, wie Hexadecyltrimethylammoniumsalzen; Cetrimoniumsalzen und Lauryldimethylbenzylammoniumsalzen, wobei nur halogenidfreie Verbindungen zum Einsatz kommen sollten. Das anionische Tensid kann ausgewählt werden aus der Gruppe von Seifen; Alkylbenzolsulfonaten; Alkansulfonaten; Olefinsulfonaten; Alkylethersulfonaten; Glycerinethersulfonaten; α-Methylestersulfonaten; Sulfofettsäuren; Alkylsulfaten; Fettalkoholethersulfaten; Glycerinethersulfaten; Fettsäureethersulfaten; Hydroxymischethersulfaten; Monoglycerid(ether)sulfaten; Fettsäureamid(ether)sulfaten; Mono- und Dialkylsulfosuccinaten; Mono- und Dialkylsulfosuccinamaten; Sulfotriglyceriden; Amidseifen; Ethercarbonsäuren und deren Salzen; Fettsäureisothionaten; Fettsäurearcosinaten; Fettsäuretauriden; N-Acylaminosäuren, wie Acyllactylaten, Acyltartraten, Acylglutamaten und Acylaspartaten; Alkyloligoglucosidsulfaten; Proteinfettsäurekondensaten, insbesondere pflanzlichen Produkten auf Weizenbasis; Alkyl(ether)phosphaten, wobei nur halogenidfreie Verbindungen zum Einsatz kommen sollten.

Erfindungsgemäß kann das Aufbringen der Kapselhülle auf die zu stabilisierende Peroxycarbonsäure, insbesondere Imidoperoxycarbonsäure, in Verfahrensschritt (e) in einer dem Fachmann an sich bekannten Vorrichtung, wie beispielsweise einem Mischer oder Kessel zum Beispiel durchgeführt werden.

Die in Verfahrensschritt (g) gegebenenfalls durchgeführte Trocknung der beladenen Gelkapseln kann durch herkömmliche Methoden erfolgen, insbesondere durch Gefriertrocknung (Lyophilisierung), Verdampfen des Dispersionsmittels, vorzugsweise bei einer Temperatur von 40 °C bis 60 °C, Ultrafiltration, Dialyse oder Sprühtrocknung unter schonenden Bedingungen.

Im Rahmen der vorliegenden Erfindung kann während bzw. nach Durchführung des Herstellungsverfahrens eine Formgebung und/oder ein Einstellen der Kapselgröße beispielsweise durch Scherung, Vertropfung, Verprillung, Pelletierung, Brikettierung, Extrudieren, Schneiden, Verrundung, Granulierung und dergleichen, vorzugsweise in einer entsprechenden Vorrichtung, vorgenommen werden.

Die nach dem erfindungsgemäßen Verfahren erhaltenen, mit mindestens einer organischen Peroxycarbonsäure, insbesondere Imidoperoxycarbonsäure, beladenen Gelkapseln können eine mittlere Größe (Durchmesser) von 0,01 mm bis 5 mm, insbesondere 0,05 mm bis 2 mm, bevorzugt 0,1 bis 1 mm, aufweisen.

Gegebenenfalls kann - falls dies anwendungsbezogen erforderlich oder gewünscht sein sollte - eine Auftrennung der Gelkapseln entsprechend ihrer Größe erfolgen, beispielsweise durch Klassieren, insbesondere Sieben.

Bei dem erfindungsgemäßen Verfahren beträgt der Gehalt an Ölphase, bezogen auf die Ölphase/Stabilisator-Gelmatrix, mehr als 80 Gew.-%, insbesondere mehr als 90 Gew.-%, bevorzugt mehr als 95 Gew.-%. Der Gehalt an Stabilisator, insbesondere Gelbildner, bezogen auf die Ölphase/Stabilisator-Gelmatrix, beträgt 0,1 Gew.-% bis 20 Gew.-%, vorzugsweise 0,3 Gew.-% bis 5 Gew.-%. Der Gehalt an organischer Peroxycarbonsäure, insbesondere Imidoperoxycarbonsäure, vorzugsweise PAP, bezogen auf die Gelkapseln, ist ≥ 30 Gew.-%, vorzugsweise ≥ 40 Gew.-% und bevorzugt ≥ 50 Gew.-%. Die Ölphase/Stabilisator-Gelmatrix macht dabei 1 bis 70 Gew.-%, bevorzugt 5 bis 50 Gew.-%, bezogen auf die Gelkapseln, aus. Je nach Anwendung sollte dabei eine Anpassung des Gehalts an Peroxycarbonsäure beispielsweise vor dem Hintergrund der Erhöhung der Handhabungssicherheit der erfindungsgemäßen Gelkapseln vorgenommen werden. So kann gegebenenfalls aus Gründen der Produktsicherheit ein allzu hoher Gehalt an Peroxycarbonsäuren nicht wünschenswert bzw. praktikabel sein. Für diese Fälle sollte der Gehalt an Peroxycarbonsäure beispielsweise 50 Gew.-%, bezogen auf die Gelkapseln, nicht überschreiten.

Die nach dem erfindungsgemäßen Verfahren hergestellten Gelkapseln weisen einen sogenannten "Controlled-released-Effekt" ("Effekt der kontrollierten Freisetzung") auf. Unter einem "Controlled-released-Effekt" ist insbesondere eine geringfügige, vorzugsweise zwischen 1 und 15 Minuten betragende, Verzögerung der Auflösung der Gelkapseln bei ihrer Anwendung, zum Beispiel in einer Waschflotte, bzw. eine Verzögerung der Freisetzung der Peroxycarbonsäure aus den erfindungsgemäßen Gelkapseln zu verstehen.

Im Rahmen des erfindungsgemäßen Verfahrens kann abschließend zusätzlich das Aufbringen einer weiteren Hülle auf die erfindungsgemäßen Gelkapseln vorgesehen sein, wodurch ein zusätzlicher stabilisierender Effekt in bezug auf die zu stabilisierende organische Peroxycarbonsäure bewirkt wird. Beispielsweise kann in dieser zusätzlichen Hülle ein Weichmacher und/oder ein Komplexbildner inkorporiert sein, der Schwermetallionen komplexieren kann, so daß eine schwermetallkatalysierte Zersetzung der Peroxycarbonsäure zumindest weitgehend unterbunden werden kann. Das Aufbringen einer zusätzlichen Hülle kann weiterhin dazu dienen, die Auflösegeschwindigkeit weiter zu modifizieren und diese in gewünschter Weise einzustellen; hierdurch kann ein zusätzlicher "Controlled-release-Effekt" bezüglich der in den erfindungsgemäßen Gelkapseln enthaltenen Peroxycarbonsäure erzielt werden. Das Aufbringen der zusätzlichen Hülle ("Coaten") kann in einer dem Fachmann an sich bekannten Weise z. B. über Wirbelschichtverfahren oder durch Adsorption des zusätzlichen Hüllmaterials ("Coatingmaterials") auf die Gelkapseln aus einer Lösung, Aufsprühen einer Lösung oder Schmelze des Hüllmaterials auf die Partikel und anschließende Verdampfung des Lösungsmittels, vorzugsweise Wasser, oder mittels Umhüllung ("Coating") im Mischer, Kessel, etc. durchgeführt werden. Dabei können erfindungsgemäß dem Fachmann an sich bekannte Materialien als zusätzliche Hüllsubstanzen eingesetzt werden, wie beispielsweise anorganische Verbindungen, z. B. Salze und anorganische Oxide, insbesondere Sulfate oder Phosphate oder aber hochmolekulare Verbindungen, wie organische Polymere, z. B. Celluloseether, Polyvinylalkohol (PVAI) oder Polyvinylpyrilidon (PVP) zum Beispiel.

Im Rahmen der vorliegenden Erfindung kann die Kapselhülle und/oder die weitere Hülle der erfindungsgemäßen Gelkapseln mindestens ein Abpuderungsmittel enthalten, wodurch die Klebrigkeit des Materials verringert und dadurch dessen Prozeßfähigkeit verbessert werden kann. Dabei sollte das Abpuderungsmittel vorzugsweise nicht alkalisch reagieren. Erfindungsgemäß bevorzugte Abpuderungsmittel sind beispielsweise Sulfatsalze und Kieselsäure, z. B. Sipemat^{®} der Firma Degussa.

Darüber hinaus kann bei dem erfindungsgemäßen Verfahren beispielsweise auf die organische Peroxycarbonsäure, vorzugsweise vor Aufbringen der Kapselhülle, eine Substanz aufgebracht werden, welche bei einer Temperatur unterhalb von 80 °C, insbesondere unterhalb von 70 °C, insbesondere mit sich selbst endotherme Reaktionen, beispielsweise Kristallwasserabspaltungsreaktionen oder Zersetzungsreaktionen, eingehen kann. Diese Substanz kann erfindungsgemäß auch mit der Peroxycarbonsäure vermengt, insbesondere vermischt sein. Eine derartige Substanz ist beispielsweise Borsäure. Im Rahmen der vorliegenden Erfindung kann diese Substanz, vorzugsweise vor Aufbringen der Kapselhülle, direkt auf die Peroxycarbonsäure aufgebracht werden, wobei beispielsweise dieselben Verfahrensschritte wie zur Bildung der weiteren Hülle eingesetzt werden können. Die zugegebene Substanz führt zu einer Erhöhung der Handhabungssicherheit der erfindungsgemäßen Gelkapseln, da sie eine gegebenenfalls auftretende Wärmetönung abfangen bzw. kompensieren kann. Unter einer Wärmetönung kann eine in den Gelkapseln lokal auftretende Temperaturerhöhung, die durch eine lokal stattfindende bzw. beginnende exotherme Zersetzung der Peroxycarbonsäure hervorgerufen werden kann, aber auch eine in einem Gebinde bzw. in der Dispersion selbst - beispielsweise bei Lagerung - auftretende Temperaturerhöhung verstanden werden. Die zugegebene Substanz, beispielsweise Borsäure, kann auch in die Gelmatrix bzw. Gelkapselhülle eingebracht werden. Erfindungsgemäß bevorzugt ist jedoch ein Aufbringen auf die Peroxycarbonsäure bzw. ein Vermengen bzw. Vermischen mit der Peroxycarbonsäure, da dies zu einer höheren Effektivität hinsichtlich der Handhabungssicherheit führt.

Die nach dem erfindungsgemäßen Verfahren in Verfahrensschritt (e) erhaltene Gelkapseldispersion bzw. die in Verfahrenschritt (f) bzw. (g) erhaltenen Gelkapseln können beispielsweise - zusammen mit weiteren Inhaltsstoffen - zu einem Wasch- oder Reinigungsmittel, insbesondere einem flüssigen Wasch- und Reinigungsmittel formuliert werden. Dabei sollte das Wasch- oder Reinigungsmittel zumindest im wesentlichen keine Halogenidionen, insbesondere keine Chloridionen, aufweisen bzw. die Menge an Halogenidionen, insbesondere Chloridionen, höchstens 500 ppm, vorzugsweise höchstens 100 ppm, besonders bevorzugt höchstens 30 ppm, betragen. Der pH-Wert sollte höchstens 7 betragen, insbesondere zwischen 3,5 und 7, vorzugsweise 4,0 und 6,5, besonders bevorzugt 4,5 und 6, liegen. Ganz besonders bevorzugt sollte der pH-Wert des Wasch- oder Reinigungsmittels etwa 5 betragen. Weiterhin kann das Wasch- oder Reinigungsmittel mindestens einen Komplexbildner enthalten; dieser kann beispielsweise ausgewählt sein aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren; diese Komplexbildner werden im Rahmen des erfindungsgemäßen Verfahrens insbesondere zur Komplexierung von Schwermetallionen eingesetzt. Weiterhin kann das Wasch- oder Reinigungsmittel gegebenenfalls mindestens ein wassermischbares Lösemittel mit einem für die organischen Peroxycarbonsäuren, insbesondere Imidoperoxycarbonsäuren, geringen Lösungsvermögen aufweisen (z. B. in Mengen von vorzugsweise mehr als 20 Gew.-%, besonders bevorzugt mehr als 30 Gew.-%, bezogen auf das Wasch- oder Reinigungsmittel) oder aber das wassermischbare Lösemittel ist das Dispersionsmittel der Dispersion. Beispielsweise kann das Lösemittel Glycerin sein, das eine geringe Löslichkeit für PAP aufweist. Das erfindungsgemäß bevorzugte Lösemittel ist jedoch Wasser. Zudem kann dem Wasch- oder Reinigungsmittel gegebenenfalls mindestens eine Katalase, mindestens eine Peroxidase und/oder mindestens ein Antioxidans zugesetzt werden. Zu weiteren Einzelheiten in bezug auf das Wasch- oder Reinigungsmittel kann auf nachfolgende Ausführungen verwiesen werden.

Das erfindungsgemäße Verfahren ist gleichermaßen ein Verfahren zur Stabilisierung von Peroxycarbonsäuren, insbesondere Imidoperoxycarbonsäuren, vorzugsweise PAP, bzw. ein Verfahren zur Erhöhung der Lagerfähigkeit von Peroxycarbonsäuren, insbesondere Imidoperoxycarbonsäuren, vorzugsweise PAP.

Gemäß einer typischen Ausführungsform kann das erfindungsgemäße Verfahren wie folgt durchgeführt werden: Zum Schutz der Peroxycarbonsäure, insbesondere Imidoperoxycarbonsäure (z. B. PAP) wird Paraffin verwendet, wobei jedoch kein Paraffinwachs mit einem höheren Schmelzpunkt eingesetzt wird, da dieses die Freisetzung von PAP im Waschprozeß einerseits nicht ausreichend schnell ermöglicht und andererseits hartnäckige Rückstände auf der Wäsche bilden kann, die sich in Form fester oder "schmieriger" Partikel auf den Textilien ablagern. Daher wird erfindungsgemäß vorteilhafterweise ein Paraffinöl eingesetzt, das bei Raumtemperatur flüssig ist. Dieses kann im Waschprozeß leicht von den anwesenden Tensiden solubilisiert werden. Um eine ausreichende mechanische Stabilität zu besitzen, sollte das Paraffinöl mit einer möglichst niedrigen Konzentration geeigneter Stabilisatoren bzw. Gelbildner geliert werden; hierzu eignen sich beispielsweise Blockcopolymere, bevorzugt solche, die aus einem steifen Block, einem flexiblen Block und wieder einem steifen Block bestehen. Beispielsweise eignen sich Polymere vom Typ Poly-(Styrol-Ethylen/Butylen-Styrol) oder Poly-(Styrol-Ethylen/Propylen-Styrol), welche unter der Bezeichnungen Kraton^{®}, z. B. Kraton^{®} G-1650, verfügbar sind. Die Herstellung der Ölphase/Stabilisator-Gelmatrix bzw. die Herstellung der Gelkapseln, in welche die Bleichmittel eingelagert sind, kann insbesondere auf folgende Weise durchgeführt werden: Zunächst wird eine Lösung des gelbildenden Polymers in Paraffinöl bei einer Temperatur oberhalb der Gelbildungstemperatur (z. B. 50 °C bis 100 °C) bereitgestellt. Diese Lösung wird auf ca. 40 °C abgekühlt und anschließend wird das Bleichmittel (Peroxycarbonsäure) darin dispergiert. Die entstandene Dispersion ("Slurry") soll dabei noch fließfähig sein, jedoch so kalt wie möglich, um eine Zersetzung der Peroxycarbonsäure zu vermeiden. Anschließend wird diese Dispersion in Wasser, dem gegebenenfalls etwas Tensid als Dispergiermittel zugesetzt sein kann, dispergiert. Durch die Mechanik der Dispergierung kann die Partikelgröße eingestellt werden. Mittels eines anschließenden, optionalen Zentrifugierens kann verkapselte bzw. mit einer Ölphase/Stabilisator-Gelmatrix versehene Peroxycarbonsäure (z. B. PAP) von etwaigem überschüssigen Paraffin abgetrennt werden. Die Partikel können dann z. B. in eine Waschmittelformulierung, bevorzugt in eine Flüssigwaschmittelformulierung, überführt werden. Im einfachsten Fall kann die Gelkapseln enthaltende Dispersion unmittelbar weiterverarbeitet werden, d. h. ohne Abtrennung der Gelkapseln.

Ein weiterer Gegenstand - gemäß einem zweiten Aspekt der vorliegenden Erfindung - sind die nach dem erfindungsgemäßen Verfahren herstellbaren, mit mindestens einer organischen Peroxycarbonsäure, insbesondere Imidoperoxycarbonsäure (z. B. PAP), beladenen Gelkapseln. Diese enthalten mindestens eine organische Peroxycarbonsäure, insbesondere Imidoperoxycarbonsäure, vorzugsweise PAP, eingelagert in eine Gelmatrix, welche als Verkapselung bzw. Beschichtung dient. Dabei umfaßt die Verkapselung bzw. Beschichtung, welche die Peroxycarbonsäure umgibt, eine gegenüber der organischen Peroxycarbonsäure inerte, durch Zugabe mindestens eines Stabilisators, insbesondere Gelbildners, verfestigte bzw. gelierte Ölphase.

In bezug auf die Natur der Ölphase und der Blockcopolymere, welche die Gelmatrix für die Peroxycarbonsäure ausbilden, kann auf obige Ausführungen zu dem erfindungsgemäßen Verfahren verwiesen werden, welche entsprechend gelten.

Die zu stabilisierende organische Peroxycarbonsäure ist insbesondere ausgewählt aus organischen Mono- und Diperoxycarbonsäuren, insbesondere Dodekandiperoxysäure, und vorzugsweise Imidoperoxysäuren, besonders bevorzugt 6-Phthalimidoperoxycapronsäure (6-Phthalimidoperoxyhexansäure, PAP). Dabei sollte die Peroxycarbonsäure bei Atmosphärendruck einen Schmelzpunkt oberhalb von 25 °C besitzen, insbesondere oberhalb von 35 °C, vorzugsweise oberhalb von 45 °C, bevorzugt oberhalb von 50 °C, besonders bevorzugt oberhalb von 100 °C.

In den erfindungsgemäßen Gelkapseln kann die Ausbildung der Gelmatrix aufgrund von physikalischen und/oder chemischen Wechselwirkungen, z. B. aufgrund physikalischer Netzwerkbildung, zwischen der Ölphase einerseits und dem Stabilisator, insbesondere Gelbildner, andererseits erfolgen.

Erfindungsgemäß ist die die zu stabilisierende Peroxycarbonsäure umgebende Verkapselung bzw. Beschichtung derart, daß die zu stabilisierende Peroxycarbonsäure zumindest im wesentlichen vollständig umschlossen ist, so daß die Peroxycarbonsäure in keinem direkten Kontakt in bezug auf das Umgebungsmilieu, insbesondere Dispersionsmittel, steht, d. h. die zu stabilisierende Peroxycarbonsäure liegt in den erfindungsgemäßen Gelkapseln vorzugsweise als Kernmaterial vor, welches von der Gelmatrix umschlossen ist.

Falls erforderlich oder anwendungsbezogen gewünscht, können dem Kernmaterial (= Peroxycarbonsäure) und/oder der Gelmatrix bzw. Gelkapselhülle noch weitere Zusatz- oder Hilfsstoffe zugegeben sein (z. B. Substanzen, die der Erhöhung der Handhabungssicherheit dienen, wie Borsäure, Stabilisatoren, Modifizierungsmittel, anorganische Salze oder Farbstoffe).

Die Gelkapseln gemäß der vorliegenden Erfindung weisen einen Gehalt an Ölphase von mehr als 80 Gew.-%, insbesondere mehr als 90 Gew.-%, bevorzugt mehr als 95 Gew.-%, auf, bezogen auf die Ölphase/Stabilisator-Gelmatrix. Der Gehalt an Stabilisator, insbesondere Gelbildner, beträgt dabei 0,1 Gew.-% bis 20 Gew.-%, vorzugsweise 0,3 Gew.-% bis 5 Gew.-%, bezogen auf die Ölphase/Stabilisator-Gelmatrix. Der Gehalt an organischer Peroxycarbonsäure, insbesondere Imidoperoxycarbonsäure, vorzugsweise PAP, beträgt ≥ 30 Gew.-%, vorzugsweise ≥ 40 Gew.-% und bevorzugt ≥ 50 Gew.-%, bezogen auf die Gelkapseln. Dabei macht die Ölphase/Stabilisator-Gelmatrix 1 bis 70 Gew.-%, bevorzugt 5 bis 50 Gew.-%, bezogen auf die Gelkapseln, aus.

Für weitere Einzelheiten zu den erfindungsgemäßen Gelkapseln kann auf obige Ausführungen zu dem erfindungsgemäßen Verfahren verwiesen werden, die hier entsprechend gelten.

Die Verwendungsmöglichkeiten der erfindungsgemäßen Gelkapseln sind sehr zahlreich und umfassend. So können die erfindungsgemäßen Gelkapseln - gemäß einem weiteren Aspekt der vorliegenden Erfindung - in Wasch- und Reinigungsmitteln, insbesondere flüssigen Wasch- und Reinigungsmitteln, Zahnpflegemitteln, Haarfärbemitteln und für Entfärbungs- bzw. Bleichmittelzusammensetzungen für technische Anwendungen eingesetzt werden.

Dabei können die erfindungsgemäßen Gelkapseln auch als "Delivery-Systeme" zur kontrollierten Freisetzung von Peroxycarbonsäuren verwendet werden, wobei die Freisetzung der Peroxycarbonsäuren insbesondere durch die Auswahl und/oder Menge der Zusammensetzung der Gelkapseln erfolgen kann. Unter Zusammensetzung wird hierbei erfindungsgemäß insbesondere die Art und/oder Menge des Blockcopolymers bzw. die Art und/oder Menge der Ölphase bzw. die Art und/oder Menge der zu verkapselnden Peroxycarbonsäuren verstanden. Hierbei kann eine Steuerung der Freisetzung der Peroxycarbonsäure insbesondere durch eine Steuerung der Glasübergangstemperaturen der Polymerblöcke des Blockcopolymers und somit über die Gelbildungstemperatur T_{gel} der Gelkapseln erfolgen. Eine weitere Modifikationsmöglichkeit stellt die Aufbringung einer zusätzlichen Hülle ("Coating") auf die erfindungsgemäßen Gelkapseln dar.

Die erfindungsgemäßen Gelkapseln können insbesondere als "Delivery-Systeme" verwendet werden, bei denen die Peroxycarbonsäuren über einen großen Zeitraum durch eine verlängerte und/oder verzögerte Freisetzung abgegeben werden ("Sustained-release-Effekt").

Ein weiterer Gegenstand der vorliegenden Erfindung - gemäß einem weiteren Aspekt der vorliegenden Erfindung - sind Wasch- und Reinigungsmittel, insbesondere flüssige Wasch- und Reinigungsmittel, Zahnpflegemittel, Haarpflegemittel, Entfärbungs- bzw. Bleichmittelzusammensetzungen für technische Anwendungen, welche die erfindungsgemäßen, mit mindestens einer organischen Peroxycarbonsäure, insbesondere Imidoperoxycarbonsäure, beladenen Gelkapseln enthalten.

Als Zahnpflegemittel können insbesondere solche Mittel angeführt werden, die zum Aufhellen bzw. Bleichen der Zähne dienen. In bezug auf Haarfärbemittel sind beispielsweise solche zu nennen, die insbesondere zum Aufhellen bzw. Färben der Haare auf Basis von Bleichmitteln eingesetzt werden. Bei den erfindungsgemäßen Entfärbungs- bzw. Bleichmittelzusammensetzungen für technische Anwendungen kann es sich beispielsweise um solche Mittel handeln, die sowohl im Hausgebrauch als auch im industriellen Bereich für diverse Anwendungen eingesetzt werden, bei denen eine gewisse Bleichleistung erforderlich ist, wie z. B. Entfernung bzw. Entfärbung von Farben und/oder Lacken oder sonstigen Verunreinigungen.

Die erfindungsgemäßen Wasch- und Reinigungsmittel, welche die erfindungsgemäßen Gelkapseln enthalten, sind sowohl im Haushalts- als auch im Industriebereich einsetzbar. Insbesondere handelt es sich bei den erfindungsgemäßen Wasch- und Reinigungsmitteln um flüssige Wasch- und Reinigungsmittel, welche die erfindungsgemäßen Gelkapseln enthalten.

Die erfindungsgemäßen Wasch- und Reinigungsmittel können zur Reinigung harter Oberflächen und/oder weicher, insbesondere textiler Oberflächen verwendet werden. Die erfindungsgemäßen Wasch- und Reinigungsmittel können insbesondere als Geschirrspülmittel, Allzweckreiniger, Badreiniger, Fußbodenreiniger, Autoreiniger, Glasreiniger, Möbelpflegemittel bzw. -reiniger, Fassadenreiniger, Waschmittel oder dergleichen verwendet werden, besonders bevorzugt als Waschmittel. Weiterhin sind die erfindungsgemäßen Wasch- und Reinigungsmittel vorzugsweise zur Reinigung von Fasern, Textilien oder Teppichen zum beispiel.

Die erfindungsgemäßen Wasch- und Reinigungsmittel enthalten neben den erfindungsgemäßen Gelkapseln an sich übliche Inhaltsstoffe bzw. Bestandteile (z. B. Tenside, Duftstoffe, Farbstoffe, Enzyme, Enzymstabilisatoren, Gerüststoffe bzw. Builder, pH-Wert-Einstellmittel, andere Bleichmittel, Bleichaktivatoren, Silberschutzmittel, schmutzabweisende Substanzen, optische Aufheller, Vergrauungsinhibitoren, Desintegrationshilfsmittel, Verdickungsmittel, Entschäumer bzw. Schauminhibitoren, Komplexbildner für Schwermetalle, schmutzabweisende Substanzen bzw. Soil-Repellents, Farbübertragungsinhibitoren, Lösungsmittel, optische Aufheller und/oder gegebenenfalls weitere übliche Inhaltsstoffe), wobei im Rahmen der vorliegenden Erfindung auf die Kompatibilität der einzelnen Inhaltsstoffe bzw. Bestandteile sowohl untereinander als auch im Hinblick auf die erfindungsgemäßen Gelkapseln bzw. die darin enthaltenen Peroxycarbonsäuren geachtet werden sollte, was durch gezielte Auswahl der Inhaltsstoffe bzw. Bestandteile und/oder ihrer jeweiligen Mengenverhältnisse realisiert wird. Auf diese Weise kann eine unerwünschte Wechselwirkung dieser Inhaltsstoffe bzw. Bestandteile mit den erfindungsgemäßen Gelkapseln bzw. den darin eingelagerten Peroxycarbonsäuren vermieden werden. Wie im folgenden noch näher ausgeführt, kann durch die gezielte Auswahl bestimmter Inhaltsstoffe bzw. Bestandteile und/oder deren Mengenverhältnisse ein stabilisierender Effekt in bezug auf die erfindungsgemäßen Gelkapseln bzw. die darin eingelagerten Peroxycarbonsäuren bewirkt werden.

Ein erfindungsgemäßes Wasch- oder Reinigungsmittel, insbesondere flüssiges Wasch- oder Reinigungsmittel, umfaßt beispielsweise die folgenden Inhaltsstoffe:
(i) mit mindestens einer organischen Peroxycarbonsäure, insbesondere Imidoperoxycarbonsäure, beladene Gelkapseln gemäß der vorliegenden Erfindung, vorzugsweise in Mengen von 0,1 Gew.-% bis 30 Gew.-%; und/oder
(ii) Tenside, insbesondere kationische und/oder anionische Tenside, vorzugsweise in Mengen von 5 bis 30 Gew.-%, und/oder nichtionische Tenside, vorzugsweise in Mengen von 0 bis 30 Gew.-%; und/oder
(iii) gegebenenfalls Elektrolyte, insbesondere anorganische und/oder organische Salze, vorzugsweise Natriumsulfat, vorzugsweise in Mengen von 0 bis 30 Gew.-%; und/oder
(iv) gegebenenfalls Komplexbildner, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren, vorzugsweise in Mengen von 0 bis 5 Gew.-%; und/oder
(v) gegebenenfalls Enzyme, wie Proteasen, Amylasen, Cellulasen und/oder Lipasen, gegebenenfalls zusammen mit Enzymstabilisatoren, vorzugsweise in Mengen von 0 bis 10 Gew.-%; und/oder
(vi) gegebenenfalls Builder, insbesondere Fettsäuren, vorzugsweise gesättigte und/oder verzweigte Fettsäuren, insbesondere mit einem Schmelzpunkt unterhalb von 30 °C, und/oder Citronensäure und/oder Citrat, vorzugsweise in Mengen von 0 bis 15 Gew.-%; und/oder
(vii) gegebenenfalls Duftstoffe, vorzugsweise in Mengen von 0 Gew.-% bis 5 Gew.-%; und/oder
(viii) gegebenenfalls Hilfsstoffe, wie Entschäumer, pH-Regulatoren, Rheologiemodifikatoren (Verdicker), Lösungsmittel, Farbstoffe; und/oder
(ix) gegebenenfalls weitere übliche Inhaltsstoffe, wie Aufheller; und/oder
(x) Wasser;
   wobei sämtliche Gewichtsangaben auf das Wasch- bzw. Reinigungsmittel bezogen sind.

Bei den erfindungsgemäßen Wasch- und Reinigungsmitteln, insbesondere flüssigen Wasch- und Reinigungsmitteln, können die Tenside in der Wasch- und Reinigungsmittelformulierung inaktiviert sein, insbesondere durch Aussalzen, d. h. Induzierung einer Phasentrennung in eine tensidarme, kontinuierliche Phase und eine vorzugsweise lamellare, im allgemeinen hochviskose, kristalline oder flüssigkristalline tensidreiche Phase, vorzugsweise durch Einbringen einer Sulfatverbindung, besonders bevorzugt Natriumsulfat, in die Wasch- oder Reinigungsmittelformulierung. Dabei wird in der Wasch- oder Reinigungsmittelformulierung insbesondere ein Auf- und/oder Anlösen der Ölphase der Gelkapseln bzw. der organischen Peroxycarbonsäure, insbesondere Imidoperoxycarbonsäure, zumindest weitgehend verhindert bzw. unterbunden. Erfindungsgemäß wird unter dem Begriff "kontinuierliche Phase" das Dispersionsmittel mit den darin gelösten Bestandteilen oder Inhaltstoffen (z. B. Salze, Tenside) verstanden. Erfindungsgemäß bevorzugt ist das Dispersionsmittel Wasser.

In diesem Zusammenhang konnte die Anmelderin zeigen, daß organische Peroxycarbonsäuren, insbesondere PAP, in Gegenwart von aktiven Tensiden (d. h. sich in freier und/oder micellarer Form in der Wasch- oder Reinigungsmittelformulierung befindlichen Tensiden) schnell zersetzt werden, da die Peroxycarbonsäuren durch die Tenside verstärkt gelöst werden und in diesem gelösten Zustand äußerst instabil sind. In diesem Zusammenhang führen insbesondere nichtionische Tenside bzw. Niotenside, beispielsweise auf Basis von Alkylpolyglykolether, zu einer beschleunigten Zersetzung der Peroxycarbonsäuren. Ohne sich auf eine Theorie festlegen zu wollen, kann die Instabilität der Peroxycarbonsäuren in Anwesenheit von Tensiden darauf zurückgeführt werden, daß Peroxycarbonsäuren im allgemeinen starke Nucleophile darstellen und unter Sauerstoffentwicklung zu den entsprechenden Carbonsäuren reduziert werden. Durch die Zugabe von Sulfat werden die Tenside zumindest teilweise inaktiviert, was insbesondere durch Aussalzen geschieht, wobei die Tenside aus der insbesondere micellaren, aktiven Form in eine vorzugsweise lamellare, kristalline oder flüssigkristalline Form (Kristall- oder Flüssigkristallbildung) überführt werden, die in einer vorzugsweise nahezu tensidfreien kontinuierlichen Phase dispergiert ist. Die dispergierten Flüssigkristalle selbst, die beispielsweise durch Zentrifugation abgetrennt werden können, sollten eine hohe Viskosität aufweisen. Der Gehalt an freien Tensiden in den erfindungsgemäßen Wasch- und Reinigungsmittelformulierungen bzw. in der kontinuierlichen Phase der Wasch- und Reinigungsmittelformulierungen sollte vorzugsweise allenfalls 1 % betragen.

Die Sulfatkonzentration in dem erfindungsgemäßen Wasch- oder Reinigungsmittel sollte derart gewählt sein, daß bei der Anwendung des Wasch- oder Reinigungsmittels in der Waschflotte die Tenside wieder in aktiver Form vorliegen, was beispielsweise durch einen Verdünnungseffekt beim Eintragen des Wasch- oder Reinigungsmittels in die Waschflotte erreicht werden kann. Insbesondere sollte die Konzentration so gewählt sein, daß - wie zuvor erwähnt - in der kontinuierlichen Phase des nichtverdünnten Wasch- oder Reinigungsmittels weniger als 1 % gelöstes Tensid vorliegt und bei Temperaturabsenkung, insbesondere bei Temperaturabsenkungen bis auf 0 °C, kein Auskristallisieren des Sulfats stattfindet.

Da insbesondere nichtionische Tenside hinsichtlich der Stabilität von Peroxycarbonsäure problematisch sein können, weisen die erfindungsgemäßen Wasch- und Reinigungsmittel ein entsprechend angepaßtes bzw. optimiertes Niotensid/geladenes Tensidverhältnis auf. Dabei sollte der Gehalt an Alkylpolyglykolethern möglichst gering sein.

Im Rahmen der vorliegenden Erfindung sollten die erfindungsgemäßen Wasch- und Reinigungsmittel, insbesondere flüssigen Wasch- und Reinigungsmittel, zumindest im wesentlichen keine Halogenidionen, insbesondere Chloridionen, aufweisen. Vorzugsweise beträgt die Menge an Halogenidionen, insbesondere Chloridionen, höchstens 500 ppm, vorzugsweise höchstens 100 ppm, besonders bevorzugt höchstens 30 ppm. Denn die Anmelderin hat überraschenderweise herausgefunden, daß eine hohe Halogenid-, insbesondere Chloridionenkonzentration, wie sie beispielsweise in herkömmlichen Wasch- und Reinigungsmitteln infolge von Verunreinigungen mancher Roh- bzw. Inhaltstoffe üblich ist, zu einem verstärkten Abbau von Peroxycarbonsäuren führt. Somit kann eine Verringerung der Halogenid-, insbesondere Chloridionenkonzentration, zu einem verminderten Abbau der Peroxycarbonsäure führen. Eine geringe Chloridionenkonzentration kann erfindungsgemäß insbesondere durch den Einsatz von Methylsulfat-, Phosphat-, Tosylat- oder Cumolsulfonatverbindungen, erreicht werden. Ferner sollten Roh- bzw. Inhaltsstoffe ausgewählt werden, die einen besonders geringen Chloridgehalt aufweisen (z. B. Verwendung von im wesentlichen halogenidfreien Komponenten, so z. B. halogenidfreien Tensiden, halogenidfreien Phosphonaten).

Weiterhin sollten die erfindungsgemäßen Wasch- und Reinigungsmittel einen pH-Wert von höchstens 7, insbesondere einen pH-Wert von 3,5 bis 7, vorzugsweise von 4,0 bis 6,5, besonders bevorzugt von 4,5 bis 6, ganz besonders bevorzugt von etwa 5, aufweisen. Denn Bleichmittel auf Basis von Peroxycarbonsäuren, wie PAP, können überraschenderweise in einer sauren Umgebung, insbesondere bei einem pH-Wert ≤ 3,5, relativ wirkungsvoll stabilisiert werden, wohingegen bei neutralen oder alkalischen pH-Werten eine relativ schnelle Zersetzung von Peroxycarbonsäuren, wie PAP, stattfindet. Die Herabsetzung des pH-Wertes in den erfindungsgemäßen Wasch- und Reinigungsmitteln kann beispielsweise durch Zugabe von Säuren oder sauren Salzen erfolgen. Bevorzugt sind Bisulfate und/oder Bicarbonate oder organische Polycarbonsäuren, die z. B. gleichzeitig auch als Buildersubstanzen eingesetzt werden können. Ferner können die als Komplexbildner eingesetzten Phosphonate als Phosphonsäuren eingearbeitet werden und anschließend der gewünschte pH-Wert durch Zugabe von Alkalien eingestellt werden.

Die erfindungsgemäßen Wasch- oder Reinigungsmittel können mindestens eine Fettsäure enthalten. Dabei sind gesättigte und/oder verzweigte Fettsäuren, insbesondere mit einem Schmelzpunkt unterhalb von 30 °C, erfindungsgemäß bevorzugt. Im Rahmen der vorliegenden Erfindung kann beispielsweise Isocarb-16^{®} der Firma Sasol in den erfindungsgemäßen Wasch- oder Reinigungsmitteln eingesetzt werden.

Die erfindungsgemäßen Wasch- oder Reinigungsmittel weisen einen optimierten Citronensäure- bzw. Citratgehalt auf. Wie die Anmelderin herausgefunden hat, kann Citronensäure bzw. Citrat zu einem Abbau von Peroxycarbonsäuren, insbesondere PAP, führen. Dennoch kann es gegebenenfalls erforderlich sein, Citronensäure bzw. Citrate in dem Wasch- oder Reinigungsmittel bzw. in dem Dispersionsmittel für die erfindungsgemäßen Gelkapseln einzusetzen (zum Beispiel als Builder und/oder als Komplexbildner). Die verwendeten Mengen sollten dabei jedoch nicht zu hoch sein und in bezug auf die Peroxycarbonsäuren, insbesondere PAP, angepaßt sein.

Zudem kann das erfindungsgemäße Wasch- oder Reinigungsmittel mindestens einen Komplexbildner enthalten, der insbesondere ausgewählt sein kann aus der Gruppe von Chinolin und/oder seinen Salzen, Alkalimetallphosphonaten, Picolinsäure oder Dipicolinsäure, Mono- oder Polyphosphonsäuren, insbesondere 1-Hydroxyethtyliden-1,2-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren. Weitere Beispiele für erfindungsgemäß verwendbare Komplexbildner für Schwermetalle sind z. B. Aminopolycarbonsäuren, Aminohydroxypolycarbonsäuren, Polyphosphonsäuren und Aminopolyphosphonsäuren. Diese Komplexbildner werden erfindungsgemäß eingesetzt, um Schwermetallionen, die insbesondere als Katalysatoren von Oxidationsprozessen fungieren und somit zu einem Abbau von Peroxycarbonsäuren, wie PAP, führen können und die beispielsweise über Wasserleitungen oder metallische Bauteile der Produktionsanlagen oder über Rohstoffe bzw. Inhaltsstoffe in das erfindungsgemäße Wasch- oder Reinigungsmittel eingetragen werden können.

Darüber hinaus können die erfindungsgemäßen Wasch- und Reinigungsmittel gegebenenfalls mindestens ein wassermischbares Lösemittel mit einem für die organische Peroxycarbonsäuren geringen Lösungsvermögen enthalten, wie vorzugsweise Glycerin.

Darüber hinaus können die erfindungsgemäßen Wasch- und Reinigungsmittel gegebenenfalls mindestens eine Katalase enthalten, um gegebenenfalls durch Umsetzung der Peroxycarbonsäure mit Wasser entstehendes Wasserstoffperoxid wirkungsvoll aus der kontinuierlichen Phase des Produktes, insbesondere der Wasch- und Reinigungsmittelformulierung, zu entfernen, so daß insbesondere dort gegebenenfalls vorhandene Enzyme wirkungsvoll vor Oxidationsprozessen, die gegebenenfalls zu einem Aktivitätsverlust der Enzyme führen können, geschützt werden. Zu diesem Zweck können den erfindungsgemäßen Wasch- oder Reinigungsmitteln gleichermaßen mindestens eine Peroxidase und/oder mindestens ein Antioxidans, gegebenenfalls zusätzlich zu der mindestens einen Katalase, zugegeben werden. Erfindungsgemäß bevorzugte Antioxidantien sind beispielsweise Ascorbinsäure, Tocopherol, Gallussäure oder deren Derivate.

Weiterhin sollte die erfindungsgemäße Wasch- oder Reinigungsmittelformulierung derart gestaltet sein, daß sie die erfindungsgemäßen Gelkapseln insbesondere im wesentlichen nicht an- bzw. auflöst. Im allgemeinen sollten die Komponenten, welche in dem erfindungsgemäßen Wasch- oder Reinigungsmittel eingesetzt werden, derart ausgewählt sein, daß sie zumindest im wesentlichen kompatibel in bezug auf die erfindungsgemäßen Gelkapseln sind, d. h. es sollten insbesondere in dem Wasch- oder Reinigungsmittel selbst, insbesondere in dem Zeitraum vor seiner Anwendung (Lagerzeit), keine unerwünschten chemischen Reaktionen, wie insbesondere Abbau-, Oxidations- bzw. Reduktionsreaktionen und/oder Hydrolysereaktionen, zwischen diesen Komponenten und den Gelkapseln auftreten, welche zu einem vorzeitigen Abbau und einem Aktivitätsverlust der Peroxycarbonsäuren führen.

Um in der Waschflotte eine ausreichende Bleichleistung zu erzielen, sollte die Peroxycarbonsäure aus den erfindungsgemäßen Gelkapseln ausreichend schnell freigesetzt werden. Die Freisetzung der Peroxycarbonsäure erfolgt bei der Anwendung des Wasch- oder Reinigungsmittels insbesondere durch physikalische bzw. physikochemische Prozesse, beispielsweise durch Solubilisieren, Emulgieren bzw. Auflösen der Ölphase/Stabilisator-Gelmatrix in der Waschflotte bzw. durch osmotische Erscheinungen bzw. Diffusionsprozesse. Gleichzeitig sollte gewährleistet sein, daß in der Wasch- oder Reinigungsformulierung selbst keine Freisetzung der Peroxycarbonsäuren erfolgt, insbesondere eine Abtrennung bzw. An- oder Auflösung der Ölphase/Stabilisator-Gelmatrix nicht stattfindet bzw. die Peroxycarbonsäure sich aufgrund der osmotischen Verhältnisse nicht löst bzw. aus den Gelkapseln diffundiert.

Die Freisetzug der Peroxycarbonsäure erfolgt durch Auflösen des Netzwerkes bzw. Gels aus Ölphase und Stabilisator, insbesondere Gelbildner, bei Anwendung der erfindungsgemäßen Gelkapseln. Wenn es sich z. B. um ein Wasch- oder Reinigungsmittelkonzentrat handelt, welches die erfindungsgemäßen Gelkapseln sowie Tenside in inaktivierter Form (z. B. durch Aussalzen beispielsweise mit Natriumsulfat bzw. in Form von Flüssigkristallen) enthält, so werden bei Verdünnung dieses Konzentrats im Rahmen der Anwendung in der Waschflotte die Tenside aus ihrer inaktivierten bzw. ausgesalzenen Form in ihre aktive Form überführt, so daß die auf diese Weise aktivierten Tenside die Gelkapselhülle bzw. Gelmatrix solubilisieren bzw. an- und/oder auflösen können. Beim Verdünnen in der Waschflotte tritt gleichzeitig ein pH-Wert-Sprung ein, so daß die Löslichkeit der Peroxycarbonsäure deutlich zunimmt. Darüber hinaus treten Diffusionsprozesse auf (z. B. Diffusion von Wassermolekülen durch die Gelkapselhülle), die gleichermaßen zu einem Auflösen der Gelkapselhülle bei der Anwendung führen. Außerdem spielen auch osmotische Vorgänge eine Rolle. Schließlich sind auch mechanische Vorgänge von Bedeutung, z. B. mechanische Zerstörung der Gelkapseln durch die in der Waschflotte befindlichen Wäschestücke bzw. durch Kontakt mit der Waschtrommel.

Wie zuvor erläutert, resultiert aus der besonderen Struktur bzw. dem besonderen Aufbau der erfindungsgemäßen Gelkapseln eine kontrollierte Freisetzung der Peroxycarbonsäure.

Die vorliegende Erfindung zeigt gegenüber dem Stand der Technik eine Reihe von Vorteilen auf:
Die Ausbildung der Ölphase/Stabilisator-Gelmatrix erfolgt bei dem erfindungsgemäßen Verfahren aufgrund von physikalisch-chemischen bzw. physikalischen Wechselwirkungen, so daß für die Ausbildung der Kapselstruktur keine Polymerisationsschritte, insbesondere radikalische Polymerisationsschritte, notwendig sind, wie dies in einigen Verfahren des Standes der Technik der Fall ist. Derartige Polymerisationen führen häufig zur Zersetzung des Aktiv- und/oder Wirkstoffs, insbesondere der empfindlichen Peroxycarbonsäure. Durch die vorliegende Erfindung wird somit ein auf die chemisch empfindlichen Peroxycarbonsäuren ausgerichtetes Verkapselungsverfahren bereitgestellt.

Des weiteren hat das erfindungsgemäße Verfahren den Vorteil, daß es mit Peroxycarbonsäuren, insbesondere Imidoperoxycarbonsäuren, beladene Gelkapseln bereitstellt, die hinsichtlich ihrer Größe und ihres Wirkstoffgehalts weit variiert bzw. maßgeschneidert werden können, so daß eine individuelle Anpassung an die jeweiligen Anforderungen, insbesondere in bezug auf die Wasch- und Reinigungsmittel, erfolgen kann. In diesem Zusammenhang ist es insbesondere vorteilhaft, daß das Verhältnis der Ölphase/Stabilisator-Gelmatrix in bezug auf den Gehalt an organischer Peroxycarbonsäure angepaßt werden kann, so daß eine Adaptation der Gelkapseln in bezug auf die Empfindlichkeit der zu verkapselnden Peroxycarbonsäure erreicht werden kann. Weiterhin ist durch die gezielte Einstellbarkeit der Gelkapselgröße eine effektive Dosierung des Wirkstoffes im Hinblick auf seine Anwendung möglich. Erfindungsgemäß kann eine geringe Menge an Ausgangssubstanz(en) für die Gelmatrix verwendet werden, so daß Gelkapseln mit einem hohen Gehalt an Peroxycarbonsäure realisiert werden können.

Im Gegensatz zu Verkapselungssystemen, die beispielsweise auf Wachsen basieren, enthalten die erfindungsgemäßen Gelkapseln keine störenden Kapselhüllen, die im Rahmen des Waschprozesses zu unerwünschten Rückständen auf der Wäsche führen.

Die erfindungsgemäßen Gelkapseln weisen den entscheidenden Vorteil auf, daß sie gegenüber Systemen des Standes der Technik über eine deutliche Erhöhung der Lagerstabilität und damit auch nach längerer Zeit noch über eine hohe Bleichaktivität verfügen.

Insbesondere eignen sich die erfindungsgemäßen Gelkapseln zur Einarbeitung bzw. Anwendung in Tenside enthaltenden Systemen, beispielsweise tensidischen (tensidhaltigen) Dispersionen für flüssige Wasch- und Reinigungsmittel. Dies ist ein besonderer Vorteil, da die unverkapselten bzw. ungeschützten Peroxycarbonsäuren, insbesondere PAP, in Gegenwart von Tensiden nicht stabil sind und rasch zersetzt werden, so daß ihr Einsatz in tensidhaltigen flüssigen, insbesondere wäßrigen Medien bislang nicht möglich bzw. allenfalls sehr beschränkt möglich war. Der stabilisierende Effekt der Gelkapseln, der zusätzlich mit einer gewünschten kontrollierten Freisetzung der verkapselten Peroxycarbonsäure verbunden ist, kann in synergistischer Weise dadurch gesteigert werden, daß das Medium, in dem sich die erfindungsgemäßen Gelkapseln befinden, derart eingestellt wird, daß es eine zusätzliche Stabilisierung in bezug auf die Peroxycarbonsäuren liefert, insbesondere durch Inaktivierung der Tenside, Optimierung bzw. Absenkung des pH-Wertes, Reduzierung des Halogenidgehaltes, Verwendung eines Lösemittels mit geringem Lösungsvermögen in bezug aus Peroxycarbonsäuren und dergleichen.

Die erfindungsgemäßen Gelkapseln lassen sich insbesondere in flüssige Wasch- und Reinigungsmittel stabil einarbeiten. Eine zusätzliche Verhinderung bzw. Verringerung von Sedimentationsprozessen kann beispielsweise durch geeignete, dem Fachmann an sich bekannte Verdickersysteme erreicht werden. Die Gelkapseln verfügen in den flüssigen Wasch- und Reinigungsmitteln über eine hohe Lagerstabilität und können auch nach größeren Zeiträumen die Peroxycarbonsäure wirkungsvoll freisetzen.

Die erfindungsgemäßen Wasch- und Reinigungsmittelformulierungen weisen aufgrund ihrer zuvor angeführten, aufeinander abgestimmten und synergistisch wirkenden Modifikationen, d. h. Anpassung der Formulierung, wie insbesondere geringer Halogenidionengehalt, Optimierung des pH-Wertes, Zugabe von Komplexbildnern, speziellen Lösemitteln bzw. Enzymen, wie Katalasen oder Peroxidasen, Zugabe von Antioxidantien, Inaktivierung der Tenside, gegenüber dem Stand der Technik erhebliche Vorteile auf, da in Verbindung mit den erfindungsgemäßen Gelkapseln der Abbau der empfindlichen Bleichmittel auf Peroxycarbonsäurebasis deutlich vermindert wird.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand der folgenden Ausführungsbeispiele verdeutlicht, welche die Erfindung jedoch keinesfalls beschränken:

### Ausführungsbeispiele:

### Beispiel 1: Herstellung von erfindungsgemäßen verkapselten bzw. beschichteten Bleichmittelpartikeln

Dünnflüssiges Paraffinöl wurde auf 70 °C erhitzt, und 2 % Kraton^{®} G-1650 wurde zugegeben. Es wurde gerührt, bis eine homogene Lösung entstanden war. Die Mischung wurde auf 40 °C abgekühlt, und es wurden 60 % homogenisiertes Eureco^{®} W, bezogen auf Paraffinöl/Kraton, unter Rühren zugegeben. Diese Mischung wurde nun in die fünffache Menge Wasser eingerührt und abgekühlt. Die so entstandene Dispersion wurde zentrifugiert. Dabei setzten sich die verkapselten bzw. beschichteten PAP-Partikel am Boden ab, während überschüssiges Paraffin aufschwamm. Die PAP-haltigen Partikel am Boden wurden entnommen, getrocknet und auf < 1 mm abgesiebt. Ihr PAP-Gehalt betrug 50 %. Sie wurden, wie in Beispiel 2 beschrieben, weiterverarbeitet. Die Herstellung der verkapselten bzw. beschichteten Bleichmittelpartikel kann in einem Mischer erfolgen. Zudem kann die Kapselhülle ein Abpuderungsmittel, wie ein Sulfatsalz, enthalten

### Beispiel 2:

Die in Beispiel 1 hergestellten verkapselten bzw. beschichteten Bleichmittelpartikel wurden zu 6 % in folgende Flüssigrezeptur eingearbeitet (die Prozentwerte sind Aktivstoffangaben):

| | |
|---|---|
| LAS (Maranil^{®} A 55 (Fa. Cognis)) | 22,5 % |
| Dehydol^{®} LT 7 (Fa. Cognis) | 4 % |
| Na₂SO₄ | 12,5 % |
| Xanthan Gum | 0,5 % |
| Sequion^{®} 10 H 60 (Fa. Polygon Chemie AG) | 1 % |
| Wasser | ad 100 % |

Der pH-Wert wurde mit Hilfe von Sequion^{®} durch Rücktitration mit Natronlauge auf 5,0 eingestellt.

### Beispiel 3:

Rezeptur für eine weitere Flüssigformulierung, in der die verkapselten bzw. beschichteten Bleichmittelpartikel eingelagert werden können:

| | |
|---|---|
| LAS (Maranil^{®} A 55 (Fa. Cognis)) | 18,5 % |
| Dehydol^{®} LT 7 (Fa. Cognis) | 8 % |
| Natriumsulfat | 11 % |
| Xanthan-Gummi | 0,4 % |
| Sequion^{®} 10 H 60 (Fa. Polygon Chemie AG) | 1 % |
| Silikon-Entschäumer | 0,2 % |
| Kapselsystem aus Beispiel 1 | 3 % |
| Wasser | ad 100 % |

### Vergleichsbeispiel:

Es wurden 3,5 % Eureco^{®} W unbehandelt in die Flüssigrezeptur gemäß Beispiel 2 eingearbeitet. Der pH-Wert betrug ebenfalls 5,0. Durch eine iodometrische Titration wurden die Erhaltungsgrade des Aktivsauerstoffs (100 % bei Lagerbeginn) nach verschiedenen Lagerzeiten bei 40 °C bestimmt.

Man erhält:

| | erfindungsgemäß | Vergleichsbeispiel |
|---|---|---|
| 1 Woche | 100,0 % | 87,5 % |
| 2 Wochen | 99,2 % | 80,0 % |

Man erkennt, daß die Stabilität von PAP mit der erfindungsgemäßen Verkapselung bzw. Beschichtung deutlich erhöht ist.

## Patentansprüche

1. Verfahren zur Herstellung von mit mindestens einer organischen Peroxycarbonsäure, insbesondere Imidoperoxycarbonsäure, beladenen Gelkapseln, **dadurch gekennzeichnet, daß** mindestens eine organische Peroxycarbonsäure, insbesondere Imidoperoxycarbonsäure, in Form fester Teilchen in eine Gelmatrix auf Basis einer gegenüber der organischen Peroxycarbonsäure inerten, durch Zugabe mindestens eines Stabilisators, insbesondere Gelbildners, verfestigten und/oder gelierten Ölphase, wobei der Schmelzpunkt der Ölphase bei Atmosphärendruck unterhalb von 35 °C liegt, eingelagert wird, insbesondere mittels einer die Peroxycarbonsäure umgebenden Verkapselung und/oder Beschichtung.

2. Verfahren zur Herstellung von mit mindestens einer organischen Peroxycarbonsäure, insbesondere Imidoperoxycarbonsäure, beladenen Gelkapseln, insbesondere nach Anspruch 1, umfassend die folgenden Verfahrensschritte:
(a) Bereitstellung einer gegenüber der organischen Peroxycarbonsäure inerten, durch Zugabe eines Stabilisators, insbesondere Gelbildners, gelierbaren und/oder verfestigbaren Ölphase, deren Schmelzpunkt bei Atmosphärendruck unterhalb von 35 °C liegt; dann
(b) Inkontaktbringen der Ölphase mit mindestens einem Stabilisator, insbesondere Gelbildner, vorzugsweise bei einer Temperatur oberhalb der Gelbildungstemperatur, so daß ein vorzugsweise flüssiges Matrixmedium auf Basis der unter Verfahrensschritt (a) bereitgestellten Ölphase und des Stabilisators, insbesondere Gelbildners, erhalten wird; dann
(c) gegebenenfalls Abkühlen des in Verfahrensschritt (b) erhaltenen Matrixmediums, vorzugsweise auf Temperaturen oberhalb der Gelbildungstemperatur des Matrixmediums; dann
(d) Einbringen mindestens einer zu stabilisierenden organischen Peroxycarbonsäure in Form fester Teilchen in das in Verfahrensschritt (b) erhaltene, gegebenenfalls in Verfahrensschritt (c) auf Temperaturen vorzugsweise oberhalb der Gelbildungstemperatur temperierte, vorzugsweise flüssige Matrixmedium, so daß eine Dispersion der festen Peroxycarbonsäure in dem vorzugsweise flüssigen Matrixmedium resultiert; dann
(e) Einbringen des in Verfahrensschritt (d) erhaltenen Matrixmediums mit den darin eingebrachten, dispergierten, festen Teilchen der zu stabilisierenden organischen Peroxycarbonsäure in ein Dispersionsmittel, insbesondere Wasser, vorzugsweise bei Temperaturen unterhalb der Gelbildungstemperatur und/oder vorzugsweise unter Zerkleinern, insbesondere durch Eintragen von Scherkräften, so daß in vorzugsweise wäßriger Dispersion befindliche Gelkapseln auf Basis von mindestens einer in eine Ölphase/Stabilisator-Gelmatrix eingelagerten oder hiermit verkapselten bzw. beschichteten organischen Peroxycarbonsäure resultieren, wobei gegebenenfalls durch das Zerkleinern, insbesondere durch das Eintragen von Scherkräften, die Gelkapselgröße gezielt eingestellt werden kann; dann
(f) gegebenenfalls Entfernen des Dispersionsmittels, insbesondere Wasser, und etwaiger überschüssiger Ölphase aus der in Verfahrensschritt (e) erhaltenen Gelkapseldispersion, insbesondere durch Zentrifugieren und/-oder Filtrieren; anschließend
(g) gegebenenfalls Trocknen der auf diese Weise erhaltenen Gelkapseln.

3. Verfahren nach Anspruch 1 oder 2, **dadurch gekennzeichnet, daß** die organische Peroxycarbonsäure ausgewählt ist aus organischen Mono- und Diperoxycarbonsäuren, insbesondere Dodekandiperoxysäure oder vorzugsweise Imidoperoxycarbonsäuren, besonders bevorzugt 6-Phthalimidoperoxycapronsäure (6-Phthalimidoperoxyhexansäure, PAP), und/oder daß die Peroxycarbonsäure bei Atmosphärendruck einen Schmelzpunkt oberhalb von 25 °C, insbesondere oberhalb von 35 °C, vorzugsweise oberhalb von 45 °C, bevorzugt oberhalb von 50 °C, besonders bevorzugt oberhalb von 100 °C, aufweist.

4. Verfahren nach einem oder mehreren der vorangehenden Ansprüche, **dadurch gekennzeichnet, daß** die gelierbare und/oder verfestigbare Ölphase ausgewählt ist aus der Gruppe von Paraffinölen, Isoparaffinölen, Silikonölen, Glyceriden, Triglyceriden, naphthalinhaltigen Ölen, kohlenwasserstoffhaltigen Lösemitteln und deren Mischungen, pflanzlichen Ölen, Rizinusöl, Maisöl, Erdnußöl, Alkylpalmitaten und Alkylalkoholbenzoaten, vorzugsweise Paraffinölen, und/oder daß die gelierbare und/oder verfestigbare Ölphase bei Atmosphärendruck einen Schmelzpunkt ≤ 30 °C, insbesondere ≤ 25 °C, vorzugsweise ≤ 20 °C, aufweist.

5. Verfahren nach einem oder mehreren der vorangehenden Ansprüche, **dadurch gekennzeichnet, daß** der Stabilisator, insbesondere Gelbildner, ein Blockcopolymer ist, insbesondere wobei das Blockcopolymer ein insbesondere hydrophobes, mit der gelierbaren und/oder verfestigbaren Ölphase unterhalb der entsprechenden Gelbildungstemperatur ein Gel, insbesondere Organogel, ausbildendes organisches Blockcopolymer ist und/oder daß der Stabilisator, insbesondere Gelbildner, ausgewählt ist aus der Gruppe von Metallseifen, Alkylhydroxybutyramiden und/oder Schichtsilikaten und/oder daß der Stabilisator, insbesondere Gelbildner, ausgewählt ist aus der Gruppe von Fettsäurederivaten, Fettalkoholen, Steroidderivaten, Anthrylderivaten, Aminosäurederivaten, Organometallverbindungen und Dibenzyliden-D-sorbitol (DBS).

6. Verfahren nach Anspruch 5, **dadurch gekennzeichnet, daß** das Blockcopolymer ein aus mindestens zwei Blöcken oder Komponenten bestehendes Polymer ist, insbesondere wobei mindestens einer der Blöcke ein harter Block und mindestens ein anderer der Blöcke ein weicher Block ist und/ oder insbesondere wobei sich die Glasübergangstemperaturen des harten und des weichen Blockes um mindestens 50 °C, insbesondere mindestens 60 °C, vorzugsweise mindestens 70 °C, unterscheiden und/oder insbesondere wobei der harte Block eine Glasübergangstemperatur T_{g(hart)} > 20 °C, insbesondere T_{g(hart)} > 50 °C, bevorzugt T_{g(hart)} > 90 °C, aufweist und/oder insbesondere wobei der weiche Block eine Glasübergangstemperatur T_{g(weich)} ≤ 20 °C, insbesondere T_{g(weich)} ≤ 0 °C, bevorzugt T_{g(weich)} ≤ - 45 °C, aufweist.

7. Verfahren nach Anspruch 5 oder 6, **dadurch gekennzeichnet, daß** mindestens ein Block des Blockcopolymers, vorzugsweise der harte Block, nicht oder nur geringfügig öllöslich ist und daß mindestens ein anderer Block des Blockcopolymers, vorzugsweise der weiche Block, öllöslich ist und/oder daß mindestens ein Block des Blockcopolymers, vorzugsweise der harte Block, schlechter öllöslich als mindestens ein anderer Block des Blockcopolymers, vorzugsweise der weiche Block, ist.

8. Verfahren nach einem der Ansprüche 5 bis 7, **dadurch gekennzeichnet, daß** der harte Block des Blockcopolymers ausgewählt ist aus der Gruppe von Polystyrolen, Poly(meth)acrylaten, Polycarbonaten, Polyestern, Polyanilinen, Poly-p-phenylenen, Polysulfonethern, Polyacrylnitrilen, Polyamiden, Polyimiden, Polyethern, Polyvinylchloriden und deren Mischungen und/oder daß der weiche Block des Blockcopolymers ausgewählt ist aus der Gruppe von Kautschuken, insbesondere gegebenenfalls substituierten Polyalkylenen, vorzugsweise Polybutadienen, und Mischungen von Kautschuken oder Polyalkylenen, wie Polybutadien-Ethylen, Polybutadien-Propylen, Polyethylen-Ethylenen; Polyvinylalkoholen; Polyalkylenglykolen, wie Polyethylenglykolen und Polypropylenglykolen; Polydimethoxysiloxanen; Polyurethanen.

9. Verfahren nach einem der Ansprüche 5 bis 7, **dadurch gekennzeichnet, daß** die mindestens zwei Blöcke oder Komponenten des Blockcopolymers jeweils vom Typ (Styrol/α-Olefin) sind, insbesondere wobei die α-Olefine der zwei Blöcke eine unterschiedliche Anzahl an Kohlenstoffatomen aufweisen und/oder insbesondere wobei das Blockcopolymer ausgewählt ist aus der Gruppe von Poly-(Styrol-Ethylen/Butylen-Styrol) und/oder Poly-(Styrol-Ethylen/Propylen-Styrol) und/oder insbesondere wobei das Blockcopolymer ein Styrol/Butadien-Blockcopolymer, Styrol/Butylen-Blockcopolymer, Styrol/Propylen-Blockcopolymer, Styrol/Butylen-Propylen-Blockcopolymer oder Styrol/Kautschuk-Blockcopolymer ist.

10. Verfahren nach einem oder mehreren der vorangehenden Ansprüche, **dadurch gekennzeichnet, daß** das Inkontaktbringen der Ölphase mit dem Stabilisator, insbesondere Gelbildner, in Verfahrensschritt (b) vorzugsweise unter Rühren und/oder bei Temperaturen oberhalb der Gelbildungstemperatur, insbesondere bei Temperaturen von 50 °C bis 100 °C, vorzugsweise von 60 °C bis 80 °C, bevorzugt bei etwa 70 °C, erfolgt und/oder daß das Abkühlen des Matrixmediums in Verfahrensschritt (c) auf Temperaturen oberhalb der Gelbildungstemperatur, insbesondere auf Temperaturen von 25 °C bis 50 °C, vorzugsweise von 35°C bis 45°C, bevorzugt auf etwa 40 °C, erfolgt.

11. Verfahren nach einem oder mehreren der vorangehenden Ansprüche, **dadurch gekennzeichnet, daß** die Teilchengröße der in Verfahrensschritt (d) eingebrachten organischen Peroxycarbonsäure ≤ 3.000 µm, insbesondere ≤ 2.500 µm, vorzugsweise ≤ 2.250 µm, bevorzugt ≤ 2.000 µm, besonders bevorzugt ≤ 1.500 µm, ist und/oder daß die Teilchengröße der organischen Peroxycarbonsäure 10 bis 3.000 µm, insbesondere 50 bis 2.500 µm, vorzugsweise 100 bis 1.500 µm, beträgt.

12. Verfahren nach einem oder mehreren der vorangehenden Ansprüche, **dadurch gekennzeichnet, daß** in Verfahrensschritt (e) die Temperatur unterhalb der Gelbildungstemperatur liegt und derart gewählt wird, daß das Matrixmedium mit den darin eingebrachten, insbesondere dispergierten, festen Teilchen der zu stabilisierenden organischen Peroxycarbonsäure noch fließfähig ist und/oder eine Zersetzung der zu stabilisierenden organischen Peroxycarbonsäure zumindest weitgehend vermieden wird.

13. Verfahren nach einem oder mehreren der vorangehenden Ansprüche, **dadurch gekennzeichnet, daß** in Verfahrensschritt (e) dem Dispersionsmittel, insbesondere Wasser, mindestens ein Dispergiermittel (Dispergator), insbesondere ein tensidisches Dispergiermittel, vorzugsweise ein kationisches und/oder anionisches Tensid, zugegeben wird, insbesondere wobei das Dispergiermittel zumindest im wesentlichen keine Halogenidionen, insbesondere keine Chloridionen, enthält, insbesondere so daß die Menge an Halogenidionen der in Verfahrensschritt (e) erhaltenen Dispersion der festen Peroxycarbonsäure in dem vorzugsweise flüssigen Matrixmedium höchstens 500 ppm, insbesondere höchstens 100 ppm, vorzugsweise höchstens 30 ppm, beträgt, und/oder insbesondere wobei das kationische Tensid ausgewählt ist aus der Gruppe von quartären Ammoniumverbindungen, wie Dimethyldistearylammoniumsalzen; Esterquats, insbesondere quaternierten Fettsäuretrialkanolaminestersalzen; Salzen langkettiger primärer Amine; quaternären Ammoniumverbindungen, wie Hexadecyltrimethylammoniumsalzen; Cetrimoniumsalzen oder Lauryldimethylbenzylammoniumsalzen, und/oder insbesondere wobei das anionische Tensid ausgewählt ist aus der Gruppe von Seifen; Alkylbenzolsulfonaten; Alkansulfonaten; Olefinsulfonaten; Alkylethersulfonaten; Glycerinethersulfonaten; □-Methylestersulfonaten; Sulfofettsäuren; Alkylsulfaten; Fettalkoholethersulfaten; Glycerinethersulfaten; Fettsäureethersulfaten; Hydroxymischethersulfaten; Monoglycerid(ether)sulfaten; Fettsäureamid(ether)sulfaten; Mono- und Dialkylsulfosuccinaten; Mono- und Dialkylsulfosuccinamaten; Sulfotriglyceriden; Amidseifen; Ethercarbonsäuren und deren Salzen; Fettsäureisothionaten; Fettsäuresarcosinaten; Fettsäuretauriden; N-Acylaminosäuren, wie Acyllactylaten, Acyltartraten, Acylglutamaten und Acylaspartaten; Alkyloligoglucosidsulfaten; Proteinfettsäurekondensaten, insbesondere pflanzlichen Produkten auf Weizenbasis; Alkyl(ether)phosphaten.

14. Verfahren nach einem oder mehreren der vorangehenden Ansprüche, **dadurch gekennzeichnet, daß** die Ausbildung der Gelmatrix in Verfahrensschritt (e) aufgrund von physikalischen und/oder chemischen Wechselwirkungen, insbesondere physikalischer Netzwerkbildung, zwischen der Ölphase einerseits und dem Stabilisator, insbesondere Gelbildner, andererseits erfolgt und/oder daß das Zerkleinern zur Einstellung der Teilchen- bzw. Gelkapselgröße in Verfahrensschritt (e) durch Eintragen von Scherkräften, Rühren, Vibration und/oder Ultraschalleintrag erfolgt.

15. Verfahren nach einem oder mehreren der vorangehenden Ansprüche, **dadurch gekennzeichnet, daß** die in Verfahrensschritt (g) gegebenenfalls durchgeführte Trocknung durch übliche Methoden erfolgt, insbesondere durch Gefriertrocknung (Lyophilisierung), Verdampfen des Dispersionsmittels, vorzugsweise bei einer Temperatur von 40 °C bis 60 °C, Ultrafiltration, Dialyse oder Sprühtrocknung unter schonenden Bedingungen.

16. Verfahren nach einem oder mehreren der vorangehenden Ansprüche, **dadurch gekennzeichnet, daß** während und/oder nach Durchführung des Herstellungsverfahrens eine Formgebung und/oder ein Einstellen der Kapselgröße insbesondere durch Scherung, Vertropfung, Verprillung, Pelletierung, Brikettierung, Extrudieren, Schneiden, Verrundung, Granulierung und dergleichen, vorzugsweise in einer entsprechenden Vorrichtung, vorgenommen wird.

17. Verfahren nach einem oder mehreren der vorangehenden Ansprüche, **dadurch gekennzeichnet, daß** die mit mindestens einer organischen Peroxycarbonsäure, insbesondere Imidoperoxycarbonsäure, beladenen Gelkapseln eine mittlere Größe (Kugeldurchmesser) von 0,01 bis 5 mm, vorzugsweise 0,05 bis 2 mm, bevorzugt 0,1 bis 1 mm, aufweisen und/oder daß der Gehalt an Ölphase mehr als 80 Gew.-%, insbesondere mehr als 90 Gew.-%, bevorzugt mehr als 95 Gew.-%, beträgt, bezogen auf die Ölphase/Stabilisator-Gelmatrix, und/oder daß der Gehalt an Stabilisator, insbesondere Gelbildner, 0,1 bis 20 Gew.-%, vorzugsweise 0,3 bis 5 Gew.-%, beträgt, bezogen auf die Ölphase/Stabilisator-Gelmatrix, und/oder daß der Gehalt an organischer Peroxycarbonsäure, insbesondere Imidoperoxycarbonsäure, vorzugsweise PAP, ≥ 30 Gew.-%, vorzugsweise ≥ 40 Gew.-%, bevorzugt ≥ 50 Gew.-%, beträgt, bezogen auf die Gelkapseln, und/oder daß die Ölphase/Stabilisator-Gelmatrix 1 bis 70 Gew.-%, bevorzugt 5 bis 50 Gew.-%, ausmacht, bezogen auf die Gelkapseln.

18. Verfahren nach einem oder mehreren der vorangehenden Ansprüche, **dadurch gekennzeichnet, daß** die in Verfahrensschritt (e) erhaltene Gelkapseldispersion und/oder die in Verfahrensschritt (f) und/oder (g) erhaltenen Gelkapseln zusammen mit weiteren Inhaltsstoffen zu einem Wasch- oder Reinigungsmittel, insbesondere einem flüssigen Wasch- oder Reinigungsmittel, formuliert werden, insbesondere wobei das Wasch- oder Reinigungsmittel:
- zumindest im wesentlichen keine Halogenidionen, insbesondere keine Chloridionen, aufweist und die Menge an Halogenidionen, insbesondere Chloridionen, höchstens 500 ppm, vorzugsweise höchstens 100 ppm, besonders bevorzugt höchstens 30 ppm, beträgt; und/oder
- einen pH-Wert von höchstens 7, insbesondere einen pH-Wert von 3,5 bis 7, vorzugsweise von 4,0 bis 6,5, besonders bevorzugt von 4,5 bis 6, ganz besonders bevorzugt von etwa 5, aufweist; und/oder
- mindestens einen Komplexbildner enthält, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren, insbesondere zur Komplexierung von Schwermetallionen; und/oder
- gegebenenfalls mindestens ein wassermischbares Lösemittel mit einem für die organische Peroxycarbonsäure, insbesondere Imidoperoxycarbonsäure, geringen Lösungsvermögen, vorzugsweise Glycerin, enthält; und/oder
- gegebenenfalls mindestens ein Enzym, insbesondere mindestens eine Katalase und/oder mindestens eine Peroxidase, vorzugsweise mindestens eine Katalase, und/oder mindestens ein Antioxidans enthält.

19. Verfahren nach einem der Ansprüche 1 bis 18 zur Herstellung von mit mindestens einer Imidoperoxycarbonsäure, vorzugsweise PAP, beladenen Gelkapseln.

20. Verfahren nach einem der Ansprüche 1 bis 19 zur Stabilisierung von Peroxycarbonsäuren, insbesondere Imidoperoxycarbonsäuren, vorzugsweise PAP, und/oder zur Erhöhung der Lagerfähigkeit von Peroxycarbonsäuren, insbesondere Imidoperoxycarbonsäuren, vorzugsweise PAP.

21. Mit mindestens einer organischen Peroxycarbonsäure, insbesondere Imidoperoxycarbonsäure, beladene Gelkapseln, erhältlich nach dem Verfahren gemäß den Ansprüchen 1 bis 20.

22. Mit mindestens einer organischen Peroxycarbonsäure, insbesondere Imidoperoxycarbonsäure, vorzugsweise PAP, beladene Gelkapseln, umfassend mindestens eine organische Peroxycarbonsäure, insbesondere Imidoperoxycarbonsäure, vorzugsweise PAP, in Form fester Teilchen, eingelagert in einer diese Teilchen enthaltenden Gelmatrix, insbesondere in Form einer die Peroxycarbonsäure umgebenden Verkapselung und/oder Beschichtung, auf Basis einer gegenüber der organischen Peroxycarbonsäure inerten, durch Zugabe mindestens eines Stabilisators, insbesondere Gelbildners, verfestigten und/oder gelierten Ölphase, wobei der Schmelzpunkt der Ölphase bei Atmosphärendruck unterhalb von 35 °C liegt.

23. Gelkapseln nach Anspruch 22, **dadurch gekennzeichnet, daß** die organische Peroxycarbonsäure ausgewählt ist aus organischen Mono- und Diperoxycarbonsäuren, insbesondere Dodekandiperoxysäure oder vorzugsweise Imidoperoxycarbonsäuren, besonders bevorzugt 6-Phthalimidoperoxycapronsäure (6-Phthalimidoperoxyhexansäure, PAP) und/oder daß die Peroxycarbonsäure bei Atmosphärendruck einen Schmelzpunkt oberhalb von 25 °C, insbesondere oberhalb von 35 °C, vorzugsweise oberhalb von 45 °C, bevorzugt oberhalb von 50 °C, besonders bevorzugt oberhalb von 100 °C, aufweist.

24. Gelkapseln nach Anspruch 22 oder 23, **dadurch gekennzeichnet, daß** die Ölphase ausgewählt ist aus der Gruppe von Paraffinölen, Isoparaffinölen, Silikonölen, Glyceriden, Triglyceriden, naphthalinhaltigen Ölen, kohlenwasserstoffhaltigen Lösemitteln und deren Mischungen, pflanzlichen Ölen, Rizinusöl, Maisöl, Erdnußöl, Alkylpalmitaten und Alkylalkoholbenzoaten, vorzugsweise Paraffinölen, und/oder daß die Ölphase bei Atmosphärendruck einen Schmelzpunkt ≤ 30 °C, insbesondere ≤ 25 °C, vorzugsweise ≤ 20 °C, aufweist.

25. Gelkapseln nach einem oder mehreren der Ansprüche 22 bis 24, **dadurch gekennzeichnet, daß** der Stabilisator, insbesondere Gelbildner, ein Blockcopolymer, insbesondere wie in den Ansprüchen 4 bis 9 definiert, ist.

26. Gelkapseln nach einem oder mehreren der Ansprüche 22 bis 25, **dadurch gekennzeichnet, daß** die Ausbildung der Gelmatrix in Verfahrensschritt (e) aufgrund von physikalischen und/oder chemischen Wechselwirkungen, insbesondere physikalischer Netzwerkbildung, zwischen der Ölphase einerseits und dem mindestens einen Stabilisator, insbesondere Gelbildner, andererseits erfolgt ist.

27. Gelkapseln nach einem oder mehreren der Ansprüche 22 bis 26, **dadurch gekennzeichnet, daß** die Teilchengröße der in Verfahrensschritt (d) eingebrachten organischen Peroxycarbonsäure ≤ 3.000 µm, insbesondere ≤ 2.500 µm, vorzugsweise ≤ 2.250 µm, bevorzugt ≤ 2.000 µm, besonders bevorzugt ≤ 1.500 µm, ist und/oder daß die Teilchengröße der organischen Peroxycarbonsäure 10 bis 3.000 µm, insbesondere 50 bis 2.500 µm, vorzugsweise 100 bis 1.500 µm, beträgt.

28. Gelkapseln nach einem oder mehreren der Ansprüche 22 bis 27, **dadurch gekennzeichnet, daß** die mit mindestens einer organischen Peroxycarbonsäure, insbesondere Imidoperoxycarbonsäure, beladenen Gelkapseln eine mittlere Größe (Kugeldurchmesser) von 0,01 mm bis 5 mm, vorzugsweise 0,05 mm bis 2 mm, bevorzugt 0,1 bis 1 mm, aufweisen.

29. Gelkapseln nach einem oder mehreren der vorangehenden Ansprüche 22 bis 28, **dadurch gekennzeichnet, daß** der Gehalt an Ölphase mehr als 80 Gew.-%, insbesondere mehr als 90 Gew.-%, bevorzugt mehr als 95 Gew.-%, beträgt, bezogen auf die Ölphase/Stabilisator-Gelmatrix, und/oder daß der Gehalt an Stabilisator, insbesondere Gelbildner, 0,1 bis 20 Gew.-%, vorzugsweise 0,3 bis 5 Gew.-%, beträgt, bezogen auf die Ölphase/Stabilisator-Gelmatrix, und/oder daß der Gehalt an organischer Peroxycarbonsäure, insbesondere Imidoperoxycarbonsäure, vorzugsweise PAP, ≥ 30 Gew.-%, vorzugsweise ≥ 40 Gew.-%, bevorzugt ≥ 50 Gew.-%, beträgt, bezogen auf die Gelkapseln, und/oder daß die Ölphase/Stabilisator-Gelmatrix 1 bis 70 Gew.-%, bevorzugt 5 bis 50 Gew.-%, bezogen auf die Gelkapseln, ausmacht.

30. Dispersionen, insbesondere wäßrige Dispersionen, enthaltend Gelkapseln nach den Ansprüchen 21 bis 29.

31. Verwendung der Gelkapseln nach den Ansprüchen 21 bis 29 und/oder der Dispersionen nach Anspruch 30 für Wasch- und Reinigungsmittel, insbesondere flüssige Wasch- und Reinigungsmittelzusammensetzungen, Zahnpflegemittel, Haarfärbemittel oder für Entfärbungs- bzw. Bleichmittelzusammensetzungen für technische Anwendungen.

32. Wasch- und Reinigungsmittel, insbesondere flüssige Wasch- und Reinigungsmittelzusammensetzungen, Zahnpflegemittel, Haarfärbemittel oder Entfärbungs- bzw. Bleichmittel für technische Anwendungen, enthaltend Gelkapseln nach den Ansprüchen 21 bis 29 und/oder Dispersionen nach Anspruch 30.

33. Wasch- oder Reinigungsmittel, insbesondere flüssiges Wasch- oder Reinigungsmittel, nach Anspruch 32, **dadurch gekennzeichnet, daß** es umfaßt:
(i) mit mindestens einer organischen Peroxycarbonsäure, insbesondere Imidoperoxycarbonsäure, beladene Gelkapseln gemäß den Ansprüchen 21 bis 29, vorzugsweise in Mengen von 0,1 bis 30 Gew.-%; und/oder
(ii) Tenside, insbesondere kationische und/oder anionische Tenside, vorzugsweise in Mengen von 5 bis 30 Gew.-%, und/oder nichtionische Tenside, vorzugsweise in Mengen von 0 bis 30 Gew.-%; und/oder
(iii) gegebenenfalls Elektrolyte, insbesondere anorganische und/oder organische Salze, vorzugsweise Natriumsulfat, vorzugsweise in Mengen von 0 bis 30 Gew.-%; und/oder
(iv) gegebenenfalls Komplexbildner, insbesondere ausgewählt aus der Gruppe von Chinolin und/oder seinen Salzen, Alkalimetall-polyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren, vorzugsweise in Mengen von 0 bis 5 Gew.-%; und/oder
(v) gegebenenfalls Enzyme, wie Proteasen, Amylasen, Cellulasen und/ oder Lipasen, gegebenenfalls zusammen mit Enzymstabilisatoren, vorzugsweise in Mengen von 0 bis 10 Gew.-%; und/oder
(vi) gegebenenfalls Builder, insbesondere Fettsäuren, vorzugsweise gesättigte und/oder verzweigte Fettsäuren, insbesondere mit einem Schmelzpunkt unterhalb von 30 °C, und/oder Citronensäure und/oder Citrat, vorzugsweise in Mengen von 0 bis 15 Gew.-%; und/oder
(vii) gegebenenfalls Duftstoffe, vorzugsweise in Mengen von 0 bis 5 Gew.-%; und/oder
(viii) gegebenenfalls Hilfsstoffe, wie Entschäumer, pH-Regulatoren, Rheologiemodifikatoren (Verdicker), Lösungsmittel, Farbstoffe; und/oder
(ix) gegebenenfalls weitere übliche Inhaltsstoffe, wie Aufheller; und/oder
(x) Wasser;
wobei sämtliche Gewichtsangaben auf das Wasch- bzw. Reinigungsmittel bezogen sind.

34. Wasch- oder Reinigungsmittel, insbesondere flüssiges Wasch- oder Reinigungsmittel, nach Anspruch 32 oder 33, **dadurch gekennzeichnet, daß** die Tenside in der Wasch- oder Reinigungsmittelformulierung inaktiviert sind, insbesondere durch Aussalzen, vorzugsweise durch Einbringen mindestens einer Sulfatverbindung, besonders bevorzugt Natriumsulfat, insbesondere so daß in der Wasch- oder Reinigungsmittelformulierung ein Auf- und/oder Anlösen der Ölphase der Gelkapseln und/oder der organischen Peroxycarbonsäure, insbesondere Imidoperoxycarbonsäure, zumindest vermindert oder verhindert ist.

35. Wasch- oder Reinigungsmittel, insbesondere flüssiges Wasch- oder Reinigungsmittel, nach den Ansprüchen 32 bis 34, **dadurch gekennzeichnet, daß** das Wasch- oder Reinigungsmittel
- zumindest im wesentlichen keine Halogenidionen, insbesondere Chloridionen, aufweist, insbesondere die Menge an Halogenidionen, insbesondere Chloridionen, höchstens 500 ppm, vorzugsweise höchstens 100 ppm, besonders bevorzugt höchstens 30 ppm, beträgt; und/oder
- einen pH-Wert von höchstens 7, insbesondere einen pH-Wert von 3,5 bis 7, vorzugsweise von 4,0 bis 6,5, besonders bevorzugt von 4,5 bis 6, ganz besonders bevorzugt von etwa 5, aufweist; und/oder
- mindestens einen Komplexbildner enthält, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren, insbesondere zur Komplexierung von Schwermetallionen; und/oder
- gegebenenfalls mindestens ein wassermischbares Lösemittel mit einem für die organische Peroxycarbonsäure, insbesondere Imidoperoxycarbonsäure, geringen Lösungsvermögen, vorzugsweise Glycerin, enthält; und/oder
- gegebenenfalls mindestens ein Enzym, insbesondere mindestens eine Katalase und/oder mindestens eine Peroxidase, vorzugsweise mindestens eine Katalase, und/oder mindestens ein Antioxidans enthält; so daß ein Abbau der Peroxycarbonsäure, insbesondere Imidoperoxycarbonsäure, vorzugsweise PAP, in dem Wasch- oder Reinigungsmittel, insbesondere flüssigem Wasch- oder Reinigungsmittel, zumindest vermindert oder verhindert wird.

## Claims

1. A process for preparing gel capsules loaded with at least one organic peroxycarboxylic acid, in particular imidoperoxycarboxylic acid, **characterized in that** at least one organic peroxycarboxylic acid, in particular imidoperoxycarboxylic acid, in the form of solid particles is incorporated, in particular by an encapsulation means and/or coating surrounding the peroxycarboxylic acid, into a gel matrix based on an oil phase which is inert to the organic peroxycarboxylic acid and is solidified and/or gelled by adding at least one stabilizer, in particular a gel former, the melting point of the oil phase being less than 35 °C at atmospheric pressure.

2. The process for preparing gel capsules loaded with at least one organic peroxycarboxylic acid, in particular imidoperoxycarboxylic acid, in particular according to claim 1, comprising the following process steps:
(a) providing an oil phase which is inert to the organic peroxycarboxylic acid, can be gelled or solidified by adding at least one stabilizer, in particular a gel former, and has a melting point of less than 35 °C at atmospheric pressure; then
(b) bringing the oil phase into contact with at least one stabilizer, in particular a gel former, preferably at a temperature above the gel formation temperature so as to obtain a preferably liquid matrix medium based on the oil phase provided in process step (a) and the stabilizer, in particular a gel former; then
(c) optionally cooling the matrix medium obtained in process step (b), preferably to temperatures above the gel formation temperature of the matrix medium; then
(d) introducing at least one organic peroxycarboxylic acid to be stabilized in the form of solid particles into the preferably liquid matrix medium obtained in process step (b) and optionally brought in process step (c) to temperatures preferably above the gel formation temperature, to form a dispersion of the solid peroxycarboxylic acid in the preferably liquid matrix medium; then
(e) introducing the matrix medium obtained in process step (d) with the dispersed solid particles of the organic peroxycarboxylic acid to be stabilized introduced therein into a dispersing agent, in particular water, preferably at temperatures of less than the gel formation temperature and/or preferably while comminuting, in particular by applying shear forces in such a way that gel capsules, based on at least one organic peroxycarboxylic acid which is incorporated in an oil phase/stabilizer gel matrix or is encapsulated or coated thereby, are formed in a preferably aqueous dispersion, it optionally being possible to deliberately adjust the gel capsule size by comminution, in particular by applying shear forces; then
(f) optionally removing the dispersing agent, in particular water, and any excess oil phase from the gel capsule dispersion obtained in process step (e), in particular by centrifugation and/or filtering; and subsequently
(g) optionally drying the gel capsules obtained in this way.

3. The process according to either claim 1 or claim 2, **characterized in that** the organic peroxycarboxylic acid is selected from organic mono- and diperoxycarboxylic acids, in particular dodecane diperoxycarboxilic acid or preferably imidoperoxycarboxylic acids, particularly preferably 6-phthalimido-peroxy-caproic acid (6-phthalimido-peroxy-hexanoic acid, PAP), and/or wherein the peroxycarboxylic acid has a melting point above 25 °C, in particular above 35 °C, preferably above 45 °C, more preferably above 50 °C and particularly preferably above 100 °C, at atmospheric pressure.

4. The process according to one or more of the preceding claims, **characterized in that** the gellable and/or solidifiable oil phase is selected from the group of paraffin oils, isoparaffin oils, silicone oils, glycerides, triglycerides, naphthalene-containing oils, hydrocarbon-containing solvents and mixtures thereof, vegetable oils, castor oil, corn oil, peanut oil, alkyl palmitates and alkyl alcohol benzoates, preferably paraffin oils, and/or wherein the gellable and/or solidifiable oil phase has a melting point ≤ 30 °C, in particular ≤ 25 °C, preferably ≤ 20 °C, at atmospheric pressure.

5. The process according to one or more of the preceding claims, **characterized in that** the stabilizer, in particular a gel former, is a block copolymer, in particular the block copolymer being an organic block copolymer which is in particular hydrophobic and forms a gel, in particular an organogel, with the gellable and/or solidifiable oil phase at less than the appropriate gel formation temperature, and/or wherein the stabilizer, in particular a gel former, is selected from the group of metal soaps, alkyl hydroxybutyramides and/or phyllosilicates, and/or wherein the stabilizer, in particular a gel former, is selected from the group of fatty acid derivatives, fatty alcohols, steroid derivatives, anthryl derivatives, amino acid derivatives, organometallic compounds and dibenzylidene-D-sorbitol (DBS).

6. The process according to claim 5, **characterized in that** the block copolymer is a polymer composed of at least two blocks or components, in particular at least one of the blocks being a hard block and at least one other block being a soft block, and/or in particular the glass transition temperatures of the hard and soft blocks differing by at least 50 °C, in particular by at least 60 °C, preferably by at least 70 °C, and/or in particular the hard block having a glass transition temperature T_{g(hard)} > 20 °C, in particular T_{g(hard)} > 50 °C, preferably T_{g(hard)} > 90 °C, and/or in particular the soft block having a glass transition temperature T_{g(soft)} ≤ 20 °C, in particular T_{g(soft)} ≤ 0 °C, more preferably T_{g(soft)} ≤ -45 °C.

7. The process according to either claim 5 or claim 6, **characterized in that** at least one block of the block copolymer, preferably the hard block, is not oil-soluble or is only slightly oil-soluble, and wherein at least one other block of the block copolymer, preferably the soft block is oil-soluble, and/or wherein at least one block of the block copolymer, preferably the hard block, is less oil-soluble than at least one other block of the block copolymer, preferably the soft block.

8. The process according to one of claims 5 to 7, **characterized in that** the hard block of the block copolymer is selected from the group of polystyrenes, poly(meth)acrylates, polycarbonates, polyesters, polyanilines, poly-p-phenylenes, polysulfone ethers, polyacrylonitriles, polyamides, polyimides, polyethers, polyvinyl chlorides and mixtures thereof, and/or wherein the soft block of the block copolymer is selected from the group of rubbers, in particular optionally substituted polyalkylenes, preferably polybutadienes, and mixtures of rubbers or polyalkylenes, such as polybutadiene-ethylene, polybutadiene-propylene, polyethylene-ethylenes; polyvinyl alcohols; polyalkylene glycols such as polyethylene glycols and polypropylene glycols; polydimethoxysiloxanes; polyurethanes.

9. The process according to one of claims 5 to 7, **characterized in that** each of the at least two blocks or components of the block copolymer are of the (styrene/α-olefin) type, in particular the α-olefins of the two blocks having a different number of carbon atoms, and/or in particular the block copolymer being selected from the group of poly(styrene-ethylene/butylene-styrene) and/or poly(styrene-ethylene/propylene-styrene), and/or in particular the block copolymer being a styrene/butadiene block copolymer, a styrene/butylene block copolymer, a styrene/propylene block copolymer, a styrene/butylene-propylene block copolymer or a styrene/rubber block copolymer.

10. The process according to one or more of the preceding claims, **characterized in that** the oil phase is brought into contact with the stabilizer, in particular a gel former, in process step (b), preferably while stirring and/or at temperatures above the gel formation temperature, in particular at temperatures of from 50 °C to 100 °C, preferably from 60 °C to 80 °C, more preferably at approximately 70 °C, and/or wherein the matrix medium is cooled in process step (c) to temperatures above the gel formation temperature, in particular to temperatures of from 25 °C to 50 °C, preferably from 35 °C to 45 °C, more preferably to approximately 40 °C.

11. The process according to one or more of the preceding claims, **characterized in that** the particle size of the organic peroxycarboxylic acid introduced in process step (d) is ≤ 3,000 µm, in particular ≤ 2,500 µm, preferably ≤ 2,250 µm, more preferably ≤ 2,000 µm, particularly preferably ≤1,500 µm, and/or wherein the particle size of the organic peroxycarboxylic acid is from 10 to 3,000 µm, in particular 50 to 2,500 µm, preferably 100 to 1,500 µm.

12. The process according to one or more of the preceding claims, **characterized in that** the temperature in process step (e) is less than the gel formation temperature and is selected in such a way that the matrix medium with the solid particles, which are introduced, in particular dispersed, therein, of the organic peroxycarboxylic acid to be stabilized is still free-flowing and/or decomposition of the organic peroxycarboxylic acid to be stabilized is at least largely avoided.

13. The process according to one or more of the preceding claims, **characterized in that** at least one dispersing agent (dispersant), in particular a surfactant dispersing agent, preferably a cationic and/or anionic surfactant, is added to the dispersing agent, in particular water, in process step (e), in particular the dispersing agent containing at least substantially no halide ions, in particular no chloride ions, in particular so the amount of halide ions in the dispersion obtained in process step (e) of the solid peroxycarboxylic acid in the preferably liquid matrix medium is at most 500 ppm in particular at most 100 ppm, preferably at most 30 ppm, and/or in particular the cationic surfactant being selected from the group of quaternary ammonium compounds such as dimethyl distearyl ammonium salts; esterquats, in particular quaternized fatty acid trialkanolamine ester salts; salts of long-chain primary amines; quaternary ammonium compounds such as hexadecyl trimethyl ammonium salts; cetrimonium salts or lauryl dimethyl benzyl ammonium salts, and/or in particular the anionic surfactant being selected from the group of soaps; alkylbenzene sulfonates; alkane sulfonates; olefin sulfonates; alkyl ether sulfonates; glycerol ether sulfonates; α-methyl ester sulfonates; sulfofatty acids; alkyl sulfates; fatty alcohol ether sulfates; glycerol ether sulfates; fatty acid ether sulfates; hydroxy mixed ether sulfates; monoglyceride (ether) sulfates; fatty acid amide (ether) sulfates; mono- and dialkyl sulfosuccinates; mono- and dialkyl sulfosuccinamates; sulfotriglycerides; amide soaps; ether carboxylic acids and the salts thereof; fatty acid isothionates; fatty acid sarcosinates; fatty acid taurides; N-acyl amino acids such as acyl lactylates, acyl tartrates, acyl glutamates, acyl aspartates; alkyl oligoglucoside sulfates; protein fatty acid condensates, in particular vegetable products based on wheat; alkyl (ether) phosphates.

14. The process according to one or more of the preceding claims, **characterized in that** the gel matrix is formed in process step (e) as a result of physical and/or chemical interactions, in particular physical network formation, between the oil phase on the one hand and the stabilizer, in particular a gel former, on the other, and/or wherein the comminution process in process step (e) for adjusting the particle or gel capsule size is carried out by applying shear forces, stirring, vibration and/or applying ultrasound.

15. The process according to one or more of the preceding claims, **characterized in that** the drying process optionally performed in process step (g) is carried out by conventional methods, in particular by freeze-drying (lyophilisation), evaporation of the dispersing agent, preferably at a temperature of from 40 to 60 °C, ultrafiltration, dialysis or spray drying, under mild conditions.

16. The process according to one or more of the preceding claims, **characterized in that** the capsule is shaped and/or the capsule size is adjusted during and/or after the preparation process has been carried out, in particular by shearing, droplet formation, prilling, pelletizing, briquetting, extrusion, cutting, rounding, granulation and the like, preferably in an appropriate device.

17. The process according to one or more of the preceding claims, **characterized in that** the gel capsules loaded with at least one organic peroxycarboxylic acid, in particular imidoperoxycarboxylic acid, have an average size (sphere diameter) of from 0.01 to 5 mm, preferably 0.05 to 2 mm, more preferably 0.1 to 1 mm, and/or wherein the oil phase content is greater than 80 % by weight, in particular greater than 90 % by weight, preferably greater than 95 % by weight, based on the oil phase/stabilizer gel matrix, and/or wherein the content of stabilizer, in particular gel former, is from 0.1 to 20 % by weight, preferably 0.3 to 5 % by weight, based on the oil phase/stabilizer gel matrix, and/or **in that** the content of organic peroxycarboxylic acid, in particular imidoperoxycarboxylic acid, preferably PAP, is ≥ 30 % by weight, preferably ≥ 40 % by weight, more preferably ≥ 50 % by weight, based on the gel capsules, and/or wherein the oil phase/stabilizer gel matrix amounts to 1 to 70 % by weight, more preferably 5 to 50 % by weight, of the gel capsules.

18. The process according to one or more of the preceding claims, **characterized in that** the gel capsule dispersion obtained in process step (e) and/or the gel capsules obtained in process steps (f) and/or (g) are formulated together with further ingredients to form a washing or cleaning agent, in particular a liquid washing or cleaning agent, in particular the washing or cleaning agent:
- containing substantially no halide ions, in particular no chloride ions and the amount of halide ions, in particular chloride ions, being at most 500 ppm, preferably at most 100 ppm, particularly preferably at most 30 ppm; and/or
- having a pH of at most 7, in particular a pH of from 3.5 to 7, preferably from 4.0 to 6.5, particularly preferably from 4.5 to 6 and most preferably of approximately 5; and/or
- containing at least one complexing agent, in particular selected from the group of quinoline and/or the salts thereof, alkali metal polyphosphonates, picolinic acid, dipicolinic acid, mono- or polyphosphonic acids, in particular 1-hydroxyethylidene-1,1-diphosphonic acid (HEDP), ethylene diamine tetraacetic acid (EDTA), diethylene triamine penta(methylene phosphonic acid) (DTPMP), azacycloheptane diphosphonate (AHP), nitrilotriacetic acid (NTA), citrate and/or short-chain dicarboxylic acids, in particular for complexing heavy metal ions; and/or
- optionally containing at least one water-miscible solvent with a low dissolving power for the organic peroxycarboxylic acid, in particular imidoperoxycarboxylic acid, preferably glycerol; and/or
- optionally containing at least one enzyme, in particular at least one catalase and/or at least one peroxidase, preferably at least one catalase, and/or at least one antioxidant.

19. Process according to one of claims 1 to 18 for preparing gel capsules loaded with at least one imidoperoxycarboxylic acid, preferably PAP.

20. Process according to one of claims 1 to 19 for stabilizing peroxycarboxylic acids, in particular imidoperoxycarboxylic acids, preferably PAP, and/or for increasing the shelf-life of peroxycarboxylic acids, in particular imidoperoxycarboxylic acid, preferably PAP.

21. Gel capsules loaded with at least one organic peroxycarboxylic acid, in particular imidoperoxycarboxylic acid, obtainable by the process according to claims 1 to 20.

22. The gel capsules loaded with at least one organic peroxycarboxylic acid, in particular imidoperoxycarboxylic acid, preferably PAP, comprising at least one organic peroxycarboxylic acid, in particular imidoperoxycarboxylic acid, preferably PAP, in the form of solid particles incorporated in a gel matrix containing these particles, in particular in the form of an encapsulation means and/or coating surrounding the peroxycarboxylic acid, based on an oil phase which is inert to the organic peroxycarboxylic acid and is solidified and/or gelled by adding at least one stabilizer, in particular a gel former, the melting point of the oil phase being less than 35 °C at atmospheric pressure.

23. The gel capsules according to claim 22, **characterized in that** the organic peroxycarboxylic acid is selected from organic mono- and diperoxycarboxylic acids, in particular dodecane diperoxy acid or preferably imidoperoxycarboxylic acids, particularly preferably 6-phthalimido-peroxy-caproic acid (6-phthalimido-peroxy-hexanoic acid, PAP), and/or wherein the peroxycarboxylic acid has a melting point above 25 °C, in particular above 35 °C, preferably above 45 °C, more preferably above 50 °C and particularly preferably above 100 °C, at atmospheric pressure.

24. The gel capsules according to either claim 22 or claim 23, **characterized in that** the oil phase is selected from the group of paraffin oils, isoparaffin oils, silicone oils, glycerides, triglycerides, naphthalene-containing oils, hydrocarbon-containing solvents and mixtures thereof, vegetable oils, castor oil, corn oil, peanut oil, alkyl palmitates and alkyl alcohol benzoates, preferably paraffin oils, and/or wherein the oil phase has a melting point ≤ 30 °C, in particular ≤ 25 °C, preferably ≤ 20 °C, at atmospheric pressure.

25. The gel capsules according to one or more of claims 22 to 24, **characterized in that** the stabilizer, in particular a gel former, is a block copolymer, in particular as defined in claims 4 to 9.

26. The gel capsules according to one or more of claims 22 to 25, **characterized in that** the gel matrix is formed in process step (e) as a result of physical and/or chemical interactions, in particular physical network formation, between the oil phase on the one hand and the at least one stabilizer, in particular a gel former, on the other.

27. The gel capsules according to one or more of claims 22 to 26, **characterized in that** the particle size of the organic peroxycarboxylic acid introduced in process step (d) is ≤ 3,000 µm, in particular ≤ 2,500 µm, preferably ≤ 2,250 µm, more preferably ≤ 2,000 µm, particularly preferably ≤1,500 µm, and/or wherein the particle size of the organic peroxycarboxylic acid is from 10 to 3,000 µm, in particular 50 to 2,500 µm, preferably 100 to 1,500 µm.

28. The gel capsules according to one or more of claims 22 to 27, **characterized in that** the gel capsules loaded with at least one organic peroxycarboxylic acid, in particular imidoperoxycarboxylic acid, have an average size (sphere diameter) of from 0.01 to 5 mm, preferably 0.05 to 2 mm, more preferably 0.1 to 1 mm.

29. The gel capsules according to one or more of claims 22 to 28, **characterized in that** the oil phase content is greater than 80 % by weight, in particular greater than 90 % by weight, more preferably greater than 95 % by weight, based on the oil phase/stabilizer gel matrix, and/or wherein the content of stabilizer, in particular gel former, is from 0.1 to 20 % by weight, preferably 0.3 to 5 % by weight, based on the oil phase/stabilizer gel matrix, and/or **in that** the content of organic peroxycarboxylic acid, in particular imidoperoxycarboxylic acid, preferably PAP, is ≥ 30 % by weight, preferably ≥ 40 % by weight, more preferably ≥ 50 % by weight, based on the gel capsules, and/or wherein the oil phase/stabilizer gel matrix amounts to 1 to 70 % by weight, more preferably 5 to 50 % by weight, of the gel capsules.

30. Dispersions, in particular aqueous dispersions containing gel capsules according to claims 21 to 29.

31. A use of the gel capsules according to claims 21 to 29 and/or the dispersion according to claim 30 for washing and cleaning agents, in particular liquid washing and cleaning agent compositions, dental care agents, hair dye agents or for decoloration or bleaching agent compositions for industrial applications.

32. Washing and cleaning agents, in particular liquid washing and cleaning agent compositions, dental care agents, hair dye agents or for decoloration or bleaching agent compositions for industrial applications, containing gel capsules according to claims 21 to 29 and/or dispersions according to claim 30.

33. The washing or cleaning agent, in particular liquid washing or cleaning agent, according to claim 32, **characterized in that** it comprises:
(i) gel capsules loaded with at least one organic peroxycarboxylic acid, in particular imidoperoxycarboxylic acid, according to claims 21 to 29, preferably in amounts of from 0.1 to 30 % by weight; and/or
(ii) surfactants, in particular cationic and/or anionic surfactants, preferably in amounts of from 5 to 30 % by weight, and/or non-ionic surfactants, preferably in amounts of from 0 to 30 % by weight; and/or
(iii) optionally electrolytes, in particular inorganic and/or organic salts, preferably sodium sulfate, preferably in amounts of from 0 to 30 % by weight; and/or
(iv) optionally complexing agents, in particular selected from the group of quinoline and/or the salts thereof, alkali metal polyphosphonates, picolinic acid, dipicolinic acid, mono- or polyphosphonic acids, in particular 1-hydroxyethylidene-1,1-diphosphonic acid (HEDP), ethylene diamine tetraacetic acid (EDTA), diethylene triamine penta(methylene phosphonic acid) (DTPMP), azacycloheptane diphosphonate (AHP), nitrilotriacetic acid (NTA), citrate and/or short-chain dicarboxylic acids, preferably in amounts of from 0 to 5 % by weight; and/or
(v) optionally enzymes, such as proteases, amylases, cellulases and/or lipases, optionally together with enzyme stabilizers, preferably in amounts of from 0 to 10 % by weight; and/or
(vi) optionally builders, in particular fatty acids, preferably saturated and/or branched fatty acids, in particular having a melting point of less than 30 °C, and/or citric acid and/or citrate, preferably in amounts of from 0 to 15 % by weight; and/or
(vii) optionally fragrances, preferably in amounts of from 0 to 5 % by weight; and/or
(viii) optionally auxiliaries, such as anti-sudsing agents, pH regulators, rheology modifiers (thickeners), solvents, dyes; and/or
(ix) optionally further optional conventional ingredients, such as brighteners; and/or
(x) water;
all the specified weights being based on the washing or cleaning agent.

34. The washing or cleaning agent, in particular liquid washing or cleaning agent, according to claim 32 or claim 33, **characterized in that** the surfactants in the washing or cleaning agent formulation are inactivated, in particular by salting-out, preferably by introducing at least one sulfate compound, particularly preferably sodium sulfate, in particular so as to at least reduce or prevent the oil phase of the gel capsules and/or the organic peroxycarboxylic acid, in particular imidoperoxycarboxylic acid, in the washing or cleaning agent formulation from dissolving or dissolving in part.

35. The washing or cleaning agent, in particular liquid washing or cleaning agent, according to claims 32 to 34, **characterized in that** the washing or cleaning agent
- contains at least substantially no halide ions, in particular chloride ions, in particular the amount of halide ions, in particular chloride ions, being at most 500 ppm, preferably at most 100 ppm, particularly preferably at most 30 ppm; and/or
- has a pH of at most 7, in particular a pH of from 3.5 to 7, preferably from 4.0 to 6.5, particularly preferably from 4.5 to 6 and most preferably of approximately 5; and/or
- contains at least one complexing agent, in particular selected from the group of quinoline and/or the salts thereof, alkali metal polyphosphonates, picolinic acid and dipicolinic acid, mono- or polyphosphonic acids, in particular 1-hydroxyethylidene-1,1-diphosphonic acid (HEDP), ethylene diamine tetraacetic acid (EDTA), diethylene triamine penta(methylene phosphonic acid) (DTPMP), azacycloheptane diphosphonate (AHP), nitrilotriacetic acid (NTA), citrate and/or short-chain dicarboxylic acids, in particular for complexing heavy metal ions; and/or
- optionally contains at least one water-miscible solvent with a low dissolving power for the organic peroxycarboxylic acid, in particular imidoperoxycarboxylic acid, preferably glycerol; and/or
- optionally contains at least one enzyme, in particular at least one catalase and/or at least one peroxidase, preferably at least one catalase, and/or at least one antioxidant;
so as to at least reduce or prevent decomposition of the peroxycarboxylic acid, in particular imidoperoxycarboxylic acid, preferably PAP, in the washing or cleaning agent, in particular a liquid washing or cleaning agent.

## Revendications

1. Procédé pour la fabrication de gélules chargées avec au moins un acide peroxycarboxylique organique, en particulier avec un acide imidoperoxycarboxylique, **caractérisé en ce qu'**on incorpore au moins un acide peroxycarboxylique, en particulier un acide imidoperoxycarboxylique sous la forme de particules solides dans une matrice sous forme de gel à base d'une phase huileuse, inerte vis-à-vis de l'acide peroxycarboxylique organique, solidifiée et/ou gélifiée par addition d'au moins un stabilisateur, en particulier d'un gélifiant, le point de fusion de la phase huileuse sous la pression atmosphérique étant inférieur à 35 °C, en particulier au moyen d'un encapsulage et/ou d'un enrobage entourant l'acide peroxycarboxylique.

2. Procédé pour la fabrication de gélules chargées avec au moins un acide peroxycarboxylique organique, en particulier avec un acide imidoperoxycarboxylique, en particulier selon la revendication 1, comprenant les étapes opératoires suivantes :
(a) de procuration d'une phase huileuse inerte vis-à-vis de l'acide peroxycarboxylique organique, apte à être gélifiée et/ou apte à être solidifiée par addition d'un stabilisateur, en particulier d'un gélifiant, le point de fusion de la phase huileuse sous la pression atmosphérique étant inférieur à 35 °C ; ensuite
(b) de mise en contact de la phase huileuse avec au moins un stabilisateur, en particulier un gélifiant, de préférence à une température supérieure à la température de gélification, si bien que l'on obtient un milieu matriciel de préférence liquide à base de la phase huileuse préparée à l'étape opératoire (a) et du stabilisateur, en particulier du gélifiant ; ensuite
(c) de refroidissement éventuel du milieu matriciel obtenu à l'étape opératoire (b), de préférence à des températures supérieures à la température de gélification du milieu matriciel ; ensuite
(d) d'incorporation d'au moins un acide peroxycarboxylique organique à stabiliser, sous la forme de particules solides, dans le milieu matriciel de préférence liquide obtenu à l'étape opératoire (b), de manière facultative maintenu, à l'étape opératoire (c), à des températures de préférence supérieures à la température de gélification si bien que l'on obtient une dispersion de l'acide peroxycarboxylique solide dans le milieu matriciel de préférence liquide ; ensuite
(e) d'incorporation du milieu matriciel obtenu à l'étape opératoire (d) comprenant les particules solides, dispersées, de l'acide peroxycarboxylique organique à stabiliser, qui y ont été incorporées, dans un agent de mise en dispersion, en particulier dans de l'eau, de préférence à des températures inférieures à la température de gélification et/ou de préférence en le soumettant à une granulation, en particulier en exerçant des forces de cisaillement, si bien que l'on obtient des gélules se trouvant de préférence en dispersion aqueuse, à base d'au moins un acide peroxycarboxylique organique incorporé dans une matrice sous forme de gel à base de phase huileuse/stabilisateur ou bien encapsulé, respectivement enduit avec ladite matrice, la dimension des gélules pouvant être réglée de manière ciblée le cas échéant via la granulation, en particulier en exerçant des forces de cisaillement ; ensuite
(f) d'élimination éventuelle de l'agent de mise en dispersion, en particulier de l'eau et de la phase huileuse éventuellement en excès, hors de la dispersion de gélule que l'on obtient à l'étape opératoire (e), en particulier par centrifugation et/ou par filtration ; et dans la foulée
(g) du séchage éventuel des gélules obtenues de cette manière.

3. Procédé selon la revendication 1 ou 2, **caractérisé en ce que** l'acide peroxycarboxylique organique est choisi parmi des acides mono- et diperoxycarboxyliques organiques, en particulier l'acide dodécanediperoxycarboxylique ou de préférence des acides imidoperoxycarboxyliques, de manière particulièrement préférée, l'acide 6-phtalimidoperoxycaproïque (acide 6-phtalimidoperoxyhexanoïque, PAP), et/ou **en ce que** l'acide peroxycarboxylique présente, sous la pression atmosphérique, un point de fusion supérieur à 25 °C, en particulier supérieur à 35 °C, de préférence supérieur à 45 °C, à titre préférentiel supérieur à 50 °C, de manière particulièrement préférée supérieur à 100 °C.

4. Procédé selon une ou plusieurs des revendications précédentes, **caractérisé en ce que** la phase huileuse apte à être gélifiée et/ou apte à être solidifiée, est choisie parmi le groupe des huiles paraffiniques, des huiles isoparaffiniques, des huiles de silicone, des glycérides, des triglycérides, des huiles naphtaléniques, des solvants à base d'hydrocarbures et leurs mélanges, des huiles végétales, de l'huile de ricin, de l'huile de maïs, de l'huile d'arachide, des palmitates d'alkyle et des benzoates d'alcools alkyliques, de préférence des huiles paraffiniques, et/ou **en ce que** la phase huileuse apte à être gélifiée et/ou apte à être solidifiée présente, sous la pression atmosphérique, un point de fusion inférieur ou égal à 30 °C, en particulier inférieur ou égal à 25 °C, de préférence inférieur ou égal à 20 °C.

5. Procédé selon une ou plusieurs des revendications précédentes, **caractérisé en ce que** le stabilisateur, en particulier le gélifiant représente un copolymère séquencé, en particulier dans lequel le copolymère séquencé représente un copolymère séquencé organique en particulier hydrophobe, formant, avec la phase huileuse apte à être gélifiée et/ou apte à être solidifiée, en dessous de la température de gélification correspondante, un gel, en particulier un organogel et/ou **en ce que** le stabilisateur, en particulier le gélifiant est choisi parmi le groupe des savons métalliques, des hydroxybutyramides d'alkyle et/ou des silicates stratifiés, et/ou **en ce que** le stabilisateur, en particulier le gélifiant est choisi parmi le groupe des dérivés d'acides gras, des alcools gras, des dérivés de stéroïdes, des dérivés d'anthryle, des dérivés d'acides aminés, des composés organométalliques et du dibenzylidène-D-sorbitol (DBS).

6. Procédé selon revendication 5, **caractérisé en ce que** le copolymère séquencé représente un polymère constitué par au moins deux séquences ou deux composants, en particulier dans lequel au moins une des séquences représente une séquence dure et au moins une autre des séquences représente une séquence molle, et/ou en particulier dans lequel les températures de transition vitreuse de la séquence dure et de la séquence molle se distinguent à concurrence d'au moins 50 °C, en particulier à concurrence d'au moins 60 °C, de préférence à concurrence d'au moins 70 °C et/ou en particulier dans lequel la séquence dure présente une température de transition vitreuse T_{g(dure)} supérieure à 20 °C, en particulier une T_{g(dure)} supérieure à 50 °C, de préférence une T_{g(dure)} supérieure à 90 °C, et/ou en particulier dans lequel la séquence molle présente une température de transition vitreuse T_{g(molle)} inférieure ou égale à 20 °C, en particulier une T_{g(molle)} inférieure ou égale à 0 °C, de préférence une T_{g(molle)} inférieure ou égale à -45 °C.

7. Procédé selon la revendication 5 ou 6, **caractérisé en ce qu'**au moins une séquence du copolymère séquencé, de préférence la séquence dure n'est pas soluble dans l'huile ou n'est que très faiblement soluble dans l'huile, et **en ce qu'**au moins une autre séquence du copolymère séquencé, de préférence la séquence molle, est soluble dans l'huile, et/ou **en ce qu'**au moins une séquence du copolymère séquencé, de préférence la séquence dure est plus difficilement soluble dans l'huile qu'au moins une autre séquence du copolymère séquencé, de préférence la séquence molle.

8. Procédé selon l'une quelconque des revendications 5 à 7, **caractérisé en ce que** la séquence dure du copolymère séquencé est choisie parmi le groupe des polystyrènes, des poly(méth)acrylates, des polycarbonates, des polyesters, des polyanilines, des poly-p-phénylènes, des polyéthersulfones, des polyacrylonitriles, des polyamides, des polyimides, des polyéthers, des chlorures de polyvinyle, et de leurs mélanges, et/ou **en ce que** la séquence molle du copolymère séquencé est choisie parmi le groupe des caoutchoucs, en particulier des polyalkylènes, de préférence de polybutadiènes éventuellement substitués, et des mélanges de caoutchoucs ou de polyalkylènes, tels que le polybutadiène-éthylène, le polybutadiène-propylène, le polyéthylène-éthylène ; des alcools polyvinyliques ; des polyalkylèneglycols tels que les polyéthylèneglycols et les polypropylèneglycols ; des polydiméthoxysiloxanes ; des polyuréthanes.

9. Procédé selon l'une quelconque des revendications 5 à 7, **caractérisé en ce que** lesdites au moins deux séquences ou lesdits au moins deux composants du copolymère séquencé sont respectivement du type (styrène/α-oléfine), en particulier dans lequel les α-oléfines des deux séquences présentent un nombre différent d'atomes de carbone, et/ou en particulier dans lequel le copolymère séquencé est choisi parmi le groupe des poly(styrène-éthylène/butylène-styrène) et/ou des poly(styrène-éthylène/propylène-styrène), et/ou en particulier dans lequel le copolymère séquencé représente un copolymère séquencé de styrène/butadiène, un copolymère séquencé de styrène/butylène, un copolymère séquencé de styrène/propylène, un copolymère séquencé de styrène/butylène-propylène ou un copolymère séquencé de styrène/caoutchouc.

10. Procédé selon une ou plusieurs des revendications précédentes, **caractérisé en ce que** la mise en contact de la phase huileuse avec le stabilisateur, en particulier le gélifiant, à l'étape (b) a lieu de préférence en agitant et/ou à des températures supérieures à la température de gélification, en particulier à des températures de 50 °C à 100 °C, de préférence de 60 °C à 80 °C, à titre préférentiel à une température d'environ 70 °C, et/ou **en ce que** le refroidissement du milieu matriciel à l'étape (c) a lieu à des températures supérieures à la température de gélification, en particulier à des températures de 25 °C à 50 °C, de préférence de 35 °C à 45 °C, à titre préférentiel à une température d'environ 40 °C.

11. Procédé selon une ou plusieurs des revendications précédentes, **caractérisé en ce que** la granulométrie de l'acide peroxycarboxylique organique incorporé à l'étape opératoire (d) est inférieure ou égale à 3000 µm, en particulier inférieure ou égale à 2500 µm, de préférence inférieure ou égale à 2250 µm, à titre préférentiel inférieure ou égale à 2000 µm, en particulier inférieure à 1500 µm, et/ou **en ce que** la granulométrie de l'acide peroxycarboxylique organique s'élève de 10 à 3000 µm, en particulier de 50 à 2500 µm, de préférence de 100 à 1500 µm.

12. Procédé selon une ou plusieurs des revendications précédentes, **caractérisé en ce que**, à l'étape opératoire (e), la température est inférieure à la température de gélification et est sélectionnée de telle sorte que le milieu matriciel, avec les particules solides, qui y ont été incorporées, en particulier mises en dispersion, de l'acide peroxycarboxylique organique à stabiliser, manifeste encore une aptitude à l'écoulement et/ou de telle sorte que l'on évite, au moins dans une large mesure, une décomposition de l'acide peroxycarboxylique organique à stabiliser.

13. Procédé selon une ou plusieurs des revendications précédentes, **caractérisé en ce que**, à l'étape opérationnelle (e), à l'agent de mise en dispersion, en particulier à de l'eau, on ajoute au moins un dispersant, en particulier à un dispersant tensioactif, de préférence un agent tensioactif cationique et/ou anionique, en particulier dans lequel le dispersant ne contient, de manière au moins essentielle, aucun ion halogénure, en particulier aucun ion chlorure, en particulier de telle sorte que la quantité des ions halogénure de la dispersion que l'on obtient à l'étape opératoire (e) de l'acide peroxycarboxylique solide dans le milieu matriciel de préférence liquide s'élève au maximum à 500 ppm, en particulier au maximum à 100 ppm, de préférence au maximum à 30 ppm, et/ou en particulier dans lequel l'agent tensioactif cationique est choisi parmi le groupe des composés d'ammonium quaternaires tels que les sels de diméthyldistéarylammonium ; des esterquats, en particulier des sels quaternisés d'esters trialcanolaminiques d'acides gras ; des sels d'amines primaires à longues chaînes ; des composés d'ammonium quaternaires tels que des sels d'hexadécyltriméthylammonium ; des sels de cétrimonium ou des sels de lauryldiméthylbenzylammonium ; et/ou en particulier dans lequel l'agent tensioactif anionique est choisi parmi le groupe des savons ; des alkylbenzènesulfonates ; des alcanesulfonates ; des oléfinesulfonates ; des sulfonates d'éthers alkyliques ; des éthersulfonates de glycérol ; des sulfonates d'esters α-méthyliques ; des acides gras sulfoniques ; des sulfates d'alkyle ; des éthersulfates d'alcools gras ; des éthersulfates de glycérol ; des éthersulfates d'acides gras ; des hydroxypolyéthersulfates ; des (éther)sulfates de monoglycérides ; des (éther)sulfates d'amides d'acides gras ; des sulfosuccinates de monoalkyle et de dialkyle ; des sulfosuccinamates de monoalkyle et de dialkyle ; des sulfotriglycérides ; des savons amidiques ; des acides éthercarboxyliques et leurs sels ; des isothionates d'acides gras ; des sarcosinates d'acides gras ; des taurides d'acides gras ; des acides N-acylaminés tels que des acyllactates, des acyltartrates, des acylglutamates et des acylaspartates ; des sulfates d'alkyloligoglucosides ; des condensats d'acides gras protéiques, en particulier des produits végétaux à base de froment ; des alkyl(éther)phosphates.

14. Procédé selon une ou plusieurs des revendications précédentes, **caractérisé en ce que** la formation de la matrice sous forme de gel à l'étape opératoire (e) a lieu sur base d'interactions physiques et/ou chimiques, en particulier d'une formation de réseau physique entre la phase huileuse d'une part et le stabilisateur, en particulier le gélifiant d'autre part, et/ou **en ce que** la granulation pour le réglage de la granulométrie respectivement de la dimension des gélules à l'étape opératoire (e) a lieu en exerçant des forces de cisaillement, par agitation, par vibration et/ou sous l'effet d'ultrasons.

15. Procédé selon une ou plusieurs des revendications précédentes, **caractérisé en ce que** le séchage éventuellement effectué à l'étape opératoire (g) a lieu via des procédés habituels, en particulier par lyophilisation, par évaporation de l'agent de mise en dispersion, de préférence à une température de 40 °C à 60 °C, par ultrafiltration, par dialyse ou par séchage par pulvérisation dans des conditions douces.

16. Procédé selon une ou plusieurs des revendications précédentes, **caractérisé en ce que**, pendant et/ou après la mise en oeuvre du procédé de fabrication, on procède à un façonnement et/ou à un réglage de la dimension des capsules, en particulier par cisaillement, par transformation en gouttes, par grenolage, par transformation en boulettes, par transformation en briquettes, par extrusion, par découpe, par formation d'un arrondi, par granulation, et analogues, de préférence dans un dispositif correspondant.

17. Procédé selon une ou plusieurs des revendications précédentes, **caractérisé en ce que** les gélules chargées avec au moins un acide peroxycarboxylique organique, en particulier un acide imidoperoxycarboxylique, présentent une dimension moyenne (diamètre de la sphère) de 0,01 à 5 mm, de préférence de 0,05 à 2 mm, à titre préférentiel de 0,1 à 1 mm, et/ou **en ce que** la teneur en phase huileuse est supérieure à 80 % en poids, en particulier supérieur à 90 % en poids, de préférence supérieure à 95 % en poids, rapportés à la matrice sous forme de gel à base de phase huileuse/stabilisateur, et/ou **en ce que** la teneur en stabilisateur, en particulier en gélifiant, s'élève de 0,1 à 20 % en poids, de préférence de 0,3 à 5 % en poids, rapportés à la matrice sous forme de gel à base de phase huileuse/stabilisateur, et/ou **en ce que** la teneur en acide peroxycarboxylique organique, en particulier en acide imidoperoxycarboxylique, de préférence en PAP, est supérieure ou égale à 30 % en poids, de préférence supérieure ou égale à 40 % en poids, de préférence supérieure ou égale à 50 % en poids, rapportés aux gélules, et/ou **en ce que** la matrice sous forme de gel à base de phase huileuse/stabilisateur représente de 1 à 70 % en poids, de préférence de 5 à 50 % en poids, rapportés aux gélules.

18. Procédé selon une ou plusieurs des revendications précédentes, **caractérisé en ce que** la dispersion des gélules que l'on obtient à l'étape opératoire (e) et/ou les gélules que l'on obtient à l'étape opératoire (f) et/ou à l'étape opératoire (g) est/sont formulées conjointement avec d'autres constituants pour obtenir un agent de lavage ou de nettoyage, de manière particulière un agent de lavage ou de nettoyage liquide, en particulier dans lequel l'agent de lavage ou de nettoyage
- ne présente, au moins à titre essentiel, aucun ion halogénure, en particulier aucun ion chlorure, la quantité des ions halogénure, en particulier des ions chlorure s'élevant au maximum à 500 ppm, de préférence au maximum à 100 ppm, de manière particulièrement préférée au maximum à 30 ppm ; et/ou
- présente une valeur de pH s'élevant au maximum à 7, en particulier une valeur de pH de 3,5 à 7, de préférence de 4,0 à 6,5, de manière particulièrement préférée de 4,5 à 6, de manière tout particulièrement préférée d'environ 5 ; et/ou
- contient au moins un formateur de complexes, en particulier choisi parmi le groupe de la quinoléine et/ou de ses sels, des polyphosphonates de métaux alcalins, de l'acide picolique et de l'acide dipicolique, des acides mono- ou polyphosphoniques, en particulier l'acide 1-hydroxyéthylidène-1,1-diphosphonique (HEDP), l'acide éthylènediaminetétracétique (EDTA), l'acide diéthylènetriaminepenta(méthylènephosphonique) (DTPMP), l'azacycloheptanediphosphonate (AHP), l'acide nitrilotriacétique (NTA) ; du citrate et/ou des acides dicarboxyliques à courtes chaînes ; en particulier pour la complexation des ions de métaux lourds ; et/ou
- contient, le cas échéant, au moins un solvant miscible à l'eau possédant un pouvoir dissolvant minime pour l'acide peroxycarboxylique organique, en particulier pour l'acide imidoperoxycarboxylique, de préférence du glycérol ; et/ou
- contient, le cas échéant, au moins une enzyme, en particulier au moins une catalase et/ou au moins une peroxydase, de préférence au moins une catalase, et/ou au moins un antioxydant.

19. Procédé selon l'une quelconque des revendications 1 à 18, pour la fabrication de gélules chargées avec au moins un acide imidoperoxycarboxylique, de préférence avec du PAP.

20. Procédé selon l'une quelconque des revendications 1 à 19, pour la stabilisation d'acides peroxycarboxyliques, en particulier d'acides imidoperoxycarboxyliques, de préférence du PAP, et/ou pour augmenter l'aptitude à l'entreposage d'acides peroxycarboxyliques, en particulier d'acides imidoperoxycarboxyliques, de préférence du PAP.

21. Gélules chargées avec au moins un acide peroxycarboxylique organique, en particulier un acide imidoperoxycarboxylique, que l'on obtient conformément aux procédés selon les revendications 1 à 20.

22. Gélules chargées avec au moins un acide peroxycarboxylique organique, en particulier un acide imidoperoxycarboxylique, à titre préférentiel avec du PAP, comprenant au moins un acide peroxycarboxylique organique, en particulier un acide imidoperoxycarboxylique, de préférence du PAP, sous la forme de particules solides, incorporé dans une matrice sous forme de gel contenant ces particules, en particulier sous la forme d'un encapsulage et/ou d'une enduction entourant l'acide peroxycarboxylique, à base d'une phase huileuse inerte vis-à-vis de l'acide peroxycarboxylique organique, solidifiée et/ou gélifiée par addition d'au moins un stabilisateur, en particulier d'un gélifiant, le point de fusion de la phase huileuse sous la pression atmosphérique étant inférieur à 35 °C.

23. Gélules selon la revendication 22, **caractérisées en ce que** l'acide peroxycarboxylique organique est choisi parmi des acides mono- et diperoxycarboxyliques organiques, en particulier l'acide dodécanediperoxycarboxylique ou de préférence des acides imidoperoxycarboxyliques, de manière particulièrement préférée, l'acide 6-phtalimidoperoxycaproïque (acide 6-phtalimidoperoxyhexanoïque, PAP), et/ou **en ce que** l'acide peroxycarboxylique présente, sous la pression atmosphérique, un point de fusion supérieur à 25 °C, en particulier supérieur à 35 °C, de préférence supérieur à 45 °C, de préférence supérieur à 50 °C, de manière particulièrement préférée supérieur à 100 °C.

24. Gélules selon la revendication 22 ou 23, **caractérisées en ce que** la phase huileuse est choisie parmi le groupe des huiles paraffiniques, des huiles isoparaffiniques, des huiles de silicone, des glycérides, des triglycérides, des huiles naphtaléniques, des solvants à base d'hydrocarbures et leurs mélanges, des huiles végétales, de l'huile de ricin, de l'huile de maïs, de l'huile d'arachide, des palmitates d'alkyle et des benzoates d'alcools alkyliques, de préférence des huiles paraffiniques, et/ou **en ce que** la phase huileuse présente, sous la pression atmosphérique, un point de fusion inférieur ou égal à 30 °C, en particulier inférieur ou égal à 25 °C, de préférence inférieur ou égal à 20 °C.

25. Gélules selon une ou plusieurs revendications 22 à 24, **caractérisées en ce que** le stabilisateur, en particulier le gélifiant représente un copolymère séquencé, en particulier tel qu'il est défini dans les revendications 4 à 9.

26. Gélules selon une ou plusieurs revendications 22 à 25, **caractérisées en ce que** la formation de la matrice sous forme de gel à l'étape opératoire (e) a lieu sur base d'interactions physiques et/ou chimiques, en particulier d'une formation de réseau physique entre la phase huileuse d'une part et ledit au moins un stabilisateur, en particulier le gélifiant d'autre part.

27. Gélules selon une ou plusieurs revendications 22 à 26, **caractérisées en ce que** la granulométrie de l'acide peroxycarboxylique organique incorporé à l'étape opératoire (d) est inférieure ou égale à 3000 µm, en particulier inférieure ou égale à 2500 µm, de préférence inférieure ou égale à 2250 µm, à titre préférentiel inférieure ou égale à 2000 µm, de manière particulièrement préférée inférieure à 1500 µm, et/ou **en ce que** la granulométrie de l'acide peroxycarboxylique organique s'élève de 10 à 3000 µm, en particulier de 50 à 2500 µm, de préférence de 100 à 1500 µm.

28. Gélules selon une ou plusieurs revendications 22 à 27, **caractérisées en ce que** les gélules chargées avec au moins un acide peroxycarboxylique organique, en particulier un acide imidoperoxycarboxylique, présentent une dimension moyenne (diamètre de la sphère) de 0,01 à 5 mm, de préférence de 0,05 à 2 mm, à titre préférentiel de 0,1 à 1 mm.

29. Gélules selon une ou plusieurs revendications 22 à 28, **caractérisées en ce que** la teneur en phase huileuse est supérieure à 80 % en poids, en particulier supérieure à 90 % en poids, de préférence supérieure à 95 % en poids, rapportés à la matrice sous forme de gel à base de phase huileuse/stabilisateur, et/ou **en ce que** la teneur en stabilisateur, en particulier en gélifiant, s'élève de 0,1 à 20 % en poids, de préférence de 0,3 à 5 % en poids, rapportés à la matrice sous forme de gel à base de phase huileuse/stabilisateur, et/ou **en ce que** la teneur en acide peroxycarboxylique organique, en particulier en acide imidoperoxycarboxylique, de préférence en PAP, est supérieure ou égale à 30 % en poids, de préférence supérieure ou égale à 40 % en poids, de préférence supérieure ou égale à 50 % en poids, rapportés aux gélules, et/ou **en ce que** la matrice sous forme de gel à base de phase huileuse/stabilisateur représente de 1 à 70 % en poids, de préférence de 5 à 50 % en poids, rapportés aux gélules.

30. Dispersions, en particulier dispersions aqueuses, contenant des gélules selon les revendications 21 à 29.

31. Utilisation des gélules selon les revendications 21 à 29 et/ou des dispersions selon la revendication 30 pour des agents de lavage et de nettoyage, en particulier pour des compositions liquides d'agents de lavage et de nettoyage, des dentifrices, des teintures capillaires ou encore pour des compositions d'agents de décoloration respectivement d'agents de blanchiment pour des applications techniques.

32. Agents de lavage et de nettoyage, en particulier compositions liquides d'agents de lavage et de nettoyage, dentifrices, teintures capillaires ou agents de décoloration respectivement agents de blanchiment pour des applications techniques contenant des gélules selon les revendications 21 à 29 et/ou des dispersions selon la revendication 30.

33. Agent de lavage et de nettoyage, en particulier agent de lavage et de nettoyage liquide, selon la revendication 32, **caractérisé en ce qu'**il comprend :
(i) des gélules chargées avec au moins un acide peroxycarboxylique organique, en particulier un acide imidoperoxycarboxylique, selon les revendications 21 à 29, de préférence dans des quantités de 0,1 à 30 % en poids ; et/ou
(ii) des agents tensioactifs, en particulier des agents tensioactifs cationiques et/ou anioniques, de préférence dans des quantités de 5 à 30 % en poids et/ou des agents tensioactifs non ioniques, de préférence dans des quantités de 0 à 30 % en poids ; et/ou
(iii) le cas échéant, des électrolytes, en particulier des sels inorganiques et/ou organiques, de préférence du sulfate de sodium, de préférence dans des quantités de 0 à 30 % en poids ; et/ou
(iv) le cas échéant, des formateurs de complexes, en particulier choisis parmi le groupe de la quinoléine et/ou de ses sels, des polyphosphonates de métaux alcalins, de l'acide picolique et de l'acide dipicolique, des acides mono- ou polyphosphoniques, en particulier l'acide 1-hydroxyéthylidène-1,1-diphosphonique (HEDP), l'acide éthylènediaminetétracétique (EDTA), l'acide diéthylènetriaminepenta(méthylènephosphonique) (DTPMP), l'azacycloheptanediphosphonate (AHP), l'acide nitrilotriacétique (NTA) ; du citrate et/ou des acides dicarboxyliques à courtes chaînes, de préférence dans des quantités de 0 à 5 % en poids ; et/ou
(v) le cas échéant, des enzymes telles que des protéases, des amylases, des cellulases et/ou des lipases, le cas échéant conjointement avec des stabilisateurs enzymatiques, de préférence dans des quantités de 0 à 10 % en poids ; et/ou
(vi) le cas échéant, des builder, en particulier des acides gras, de préférence des acides gras saturés et/ou ramifiés, en particulier possédant un point de fusion inférieure à 30 °C, et/ou de l'acide citrique et/ou du citrate, de préférence dans des quantités de 0 à 15 % en poids ; et/ou
(vii) le cas échéant, des parfums, de préférence dans des quantités de 0 à 5 % en poids ; et/ou
(viii) le cas échéant, des adjuvants tels que des agents antimousse, des régulateurs du pH, des modificateurs de la rhéologie (des épaississants), des solvants, des colorants ; et/ou
(ix) le cas échéant, d'autres constituants habituels tels que des agents de blanchiment optique ; et/ou
(x) de l'eau ;
l'ensemble des indications pondérales se rapportant à l'agent de lavage respectivement de nettoyage.

34. Agent de lavage ou de nettoyage, en particulier agent de lavage ou de nettoyage liquide, selon la revendication 32 ou 33, **caractérisé en ce que** les agents tensioactifs dans la formulation d'agent de lavage ou de nettoyage sont inactivés, en particulier par relargage, de préférence par incorporation d'au moins un composé de sulfate, de manière particulièrement préférée du sulfate de sodium, en particulier de telle sorte que, dans la formulation de l'agent de lavage ou de nettoyage, à tout le moins on diminue, voire on empêche une dissolution ou une dissolution naissante de la phase huileuse des gélules et/ou de l'acide peroxycarboxylique organique, en particulier de l'acide imidoperoxycarboxylique.

35. Agent de lavage ou de nettoyage, en particulier agent de lavage ou de nettoyage liquide, selon les revendications 32 à 34, **caractérisé en ce que** l'agent de lavage ou de nettoyage :
- ne présente, de manière au moins essentielle, aucun ion halogénure, en particulier aucun ion chlorure, de manière particulière la quantité des ions halogénure, en particulier des ions chlorure s'élevant au maximum à 500 ppm, de préférence au maximum à 100 ppm, de manière particulièrement préférée au maximum à 30 ppm ; et/ou
- présente une valeur de pH s'élevant au maximum à 7, en particulier une valeur de pH de 3,5 à 7, de préférence de 4,0 à 6,5, de manière particulièrement préférée de 4,5 à 6, de manière tout particulièrement préférée d'environ 5 ; et/ou
- contient au moins un formateur de complexes, en particulier choisi parmi le groupe de la quinoléine et/ou de ses sels, des polyphosphonates de métaux alcalins, de l'acide picolique et de l'acide dipicolique, des acides mono- ou polyphosphoniques, en particulier l'acide 1-hydroxyéthylidène-1,1-diphosphonique (HEDP), l'acide éthylènediaminetétracétique (EDTA), l'acide diéthylènetriaminepenta(méthylènephosphonique) (DTPMP), l'azacycloheptanediphosphonate (AHP), l'acide nitrilotriacétique (NTA) ; du citrate et/ou des acides dicarboxyliques à courtes chaînes ; en particulier pour la complexation des ions de métaux lourds ; et/ou
- contient, le cas échéant, au moins un solvant miscible à l'eau possédant un pouvoir dissolvant minime pour l'acide peroxycarboxylique organique, en particulier pour l'acide imidoperoxycarboxylique, de préférence du glycérol ; et/ou
- contient, le cas échéant, au moins une enzyme, en particulier au moins une catalase et/ou au moins une peroxydase, de préférence au moins une catalase, et/ou au moins un antioxydant ;
- de telle sorte que, dans l'agent de lavage ou de nettoyage, en particulier dans l'agent de lavage ou de nettoyage liquide, à tout le moins on diminue, voire on empêche une décomposition de l'acide peroxycarboxylique organique, en particulier de l'acide imidoperoxycarboxylique, de préférence du PAP.
